(19) **Europäisches Patentamt**
**European Patent Office**
**Office européen des brevets**

(11) **EP 1 807 002 B1**

(12) **FASCICULE DE BREVET EUROPEEN**

(45) Date de publication et mention
de la délivrance du brevet:
**20.02.2013 Bulletin 2013/08**

(21) Numéro de dépôt: **05819328.5**

(22) Date de dépôt: **03.11.2005**

(51) Int Cl.:
***A61B 5/0205*** (2006.01)

(86) Numéro de dépôt international:
**PCT/FR2005/050928**

(87) Numéro de publication internationale:
**WO 2006/048587 (11.05.2006 Gazette 2006/19)**

(54) **DISPOSITIF DE PREDICTION D'EVENEMENTS MEDICAUX ANORMAUX ET/OU D'AIDE AU DIAGNOSTIC ET/OU DE MONITORAGE POUR LA DETERMINATION DE LA PROFONDEUR D'ANESTHESIE**

VORRICHTUNG ZUR VORHERSAGE ANOMALER MEDIZINISCHER EREIGNISSE UND/ODER ZUR UNTERSTÜTZUNG DER DIAGNOSE UND/ODER ÜBERWACHUNG ZUR BESTIMMUNG DER TIEFE EINER ANÄSTHESIE

DEVICE FOR PREDICTING ABNORMAL MEDICAL EVENTS AND/OR ASSISTING IN DIAGNOSIS AND/OR MONITORING IN ORDER TO DETERMINE DEPTH OF ANAESTHESIA

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **03.11.2004 FR 0411726**

(43) Date de publication de la demande:
**18.07.2007 Bulletin 2007/29**

(73) Titulaires:
• **Quintin, Luc**
**69008 Lyon (FR)**
• **Cividjian, Andrei**
**69450 St. Cyr au Mont d'Or (FR)**

(72) Inventeurs:
• **Quintin, Luc**
**69008 Lyon (FR)**
• **Cividjian, Andrei**
**69450 St. Cyr au Mont d'Or (FR)**

(74) Mandataire: **Cabinet Plasseraud**
**52, rue de la Victoire**
**75440 Paris Cedex 09 (FR)**

(56) Documents cités:
**WO-A-2004/034897     FR-A- 2 747 027
US-A- 4 788 982**

• **LEGRAMANTE JACOPO M ET AL: "Investigating feed-forward neural regulation of circulation from analysis of spontaneous arterial pressure and heart rate fluctuations" CIRCULATION, vol. 99, no. 13, 6 avril 1999 (1999-04-06), pages 1760-1766, XP002334802 ISSN: 0009-7322 cité dans la demande**
• **GRATZE G ET AL: "A software package for non-invasive, real-time beat-to-beat monitoring of stroke volume, blood pressure, total peripheral resistance and for assessment of autonomic functionAn updated and improved software version for Windows 95/NT and the complete biosignal electronics (ECG, ICG, beat-to-beat and o" COMPUTERS IN BIOLOGY AND MEDICINE, NEW YORK, NY, US, vol. 28, no. 2, 1 juin 1998 (1998-06-01), pages 121-141, XP004532373 ISSN: 0010-4825 cité dans la demande**

## Description

Domaine de l'invention

Le domaine de l'invention couvre le domaine de l'assistance technologique pour les praticiens en anesthésie ou en soins intensifs, ainsi que le domaine du monitorage médical simple et fiable de gestion de l'anesthésie et/ou de prédiction d'événements médicaux délétères. Les buts visés sont notamment l'anticipation des soins nécessaires aux patients et/ou le maintien des patients dans l'état anesthésique adéquat pour l'intervention chirurgicale à l'instant considéré.

Plus précisément l'invention concerne un procédé et un dispositif de prédiction d'évènements médicaux anormaux et/ou d'aide au diagnostic et/ou de monitorage, en particulier pour la détermination de la profondeur d'anesthésie.

L'invention vise également les algorithmes et les programmes d'ordinateur pour la mise en oeuvre de ce procédé.

Contexte de l'invention et Problème technique

L'anesthésie générale peut être définie comme un état d'inconscience induit pharmacologiquement, dans lequel le patient ne perçoit pas (analgésie) et ne se souvient pas (amnésie) de la stimulation douloureuse. Une telle anesthésie peut être considérée comme "adéquate" ou suffisamment "profonde". L'anesthésie a au moins deux composantes: d'une part la composante hypnotique qui assure la perte de conscience et l'amnésie et d'autre part la composante analgésique. Malgré le grand nombre d'anesthésies générales qui ont lieu annuellement dans le monde (plus de 50 millions dans les pays développés), des grands progrès restent encore à faire pour améliorer la sécurité des patients et le travail des spécialistes.

En effet, pour assurer l'adéquation de l'anesthésie, la dose d'anesthésique doit être ajustée en permanence, variant selon la variabilité biologique du patient et selon l'intensité de la stimulation douloureuse. Actuellement, l'ajustement de la dose d'anesthésique est effectué selon des signes cliniques traditionnels qui ne sont pas suffisamment fiables: hypertension, tachycardie, larmoiement, réponse motrice à la stimulation douloureuse, etc. De ce fait, un moniteur de la profondeur de l'anesthésie serait nécessaire pour un dosage précis de l'anesthésique, évitant ainsi le risque de sur- ou sous-dosage.

Un sous-dosage anesthésique entraîne un risque de réveil intra-opératoire inopiné. Dans toute chirurgie fine, des mouvements intra-opératoires inopinés peuvent entraîner des conséquences délétères. De plus, un réveil intra-opératoire inopiné présente un risque de mémorisation et de névrose post-traumatique.

Un surdosage anesthésique augmente les risques cardiovasculaires ou ventilatoires ainsi que le temps d'émergence. Ceci ralentit la rotation des salles d'opération et de réveil.

Des moniteurs de la composante hypnotique de l'anesthésie existent actuellement. Ces moniteurs sont fondés soit sur l'électroencéphalogramme (EEG) soit sur les potentiels évoqués auditifs. Grâce à ces moniteurs qui enregistrent essentiellement les zones du cortex cérébral, le taux de mémorisation intra-opératoire peut être considérablement réduit. Toutefois, le problème du mouvement intra-opératoire inopiné reste non-résolu à ce jour. En effet, le mouvement intra-opératoire inopiné est plus vraisemblablement lié à la composante analgésique qu'à la composante hypnotique de l'anesthésie. Les signaux cardiovasculaires pourraient être des témoins de l'activité des zones sous-corticales (hypothalamus, matière grise péri-aquéductale, centre vasomoteur) qui pourraient recevoir et générer des signes plus précoces de l'allégement de l'anesthésie que les zones corticales. Dans ce contexte, un moniteur de l'allégement de l'anesthésie fondé sur des signaux cardiovasculaires, serait d'un grand intérêt notamment pour la prédiction du mouvement intra opératoire inopiné.

Au-delà de ce problème précis de mouvement inopiné, il existe un besoin constant de procédés et de dispositifs :

- o d'aide au diagnostic en temps réel en soins intensifs de cardiologie ou en cardiologie ambulatoire (prévision de l'ischémie myocardique ou de la fibrillation ventriculaire),
- o de monitorage de l'anesthésie pour d'autres facteurs que la profondeur d'anesthésie et la prédiction du réveil (ischémie myocardique postopératoire, anesthésie locorégionale, douleur, etc...),
- o de réanimation médicale ou chirurgicale (adulte, pédiatrique, néonatalogie),
- o d'obstétrique,
- o de médecine d'urgence/accueil, oxyologie,
- o de médecine spatiale,
- o d'exploration fonctionnelle du système nerveux autonome (syncope, diabète, dystonie neurovégétative...).

Les moyens d'assistance attendus par les praticiens devraient leur permettre d'être avertis suffisamment tôt de la survenue de phénomènes anormaux chez le patient, afin de parer efficacement à ces phénomènes et à leurs conséquences.

Il est souhaitable que les données transmises aux praticiens, soient simples et faciles à lire, à interpréter et à exploiter.

Etat de la technique

On connaît un procédé et un dispositif de monitorage dénommé TASK-FORCE MONITOR 3040, développé et commercialisé par la Société autrichienne CNSystems. Le TASK-FORCE MONITOR comprend une partie logiciel (software) et une partie matériel (hardware). Cette dernière inclut des capteurs de signaux et un ordinateur équipé d'un écran d'affichage présentant jusqu'à 14 paramètres.

Ce procédé et ce dispositif antérieurs sont décrits notamment dans l'article de « Gratze et al., Computers in Biology and Medicine, 1998, vol.28, 121-142 ». Ce logiciel et ses périphériques visent à assurer un monitorage non-invasif en temps réel et battement-après-battement, du volume d'éjection systolique, de la pression sanguine et de l'index de résistance périphérique totale. Un objectif visé par ce système est l'évaluation de l'activité du système nerveux autonome qui contrôle le fonctionnement cardiaque.

Le dispositif TASK-FORCE MONITOR comprend des capteurs de mesure de la pression sanguine mesurée à l'aide d'un brassard (DINAMAP®), de la pression artérielle mesurée au doigt (FINAPRES®), de l'électrocardiogramme ECG, de l'impédance du cardiogramme ICG et du phonocardiogramme PCG.

Le logiciel met en oeuvre des algorithmes de calcul de l'intervalle RR entre 2 complexes QRS de l'ECG, de la pression artérielle systolique PAS, de la pression artérielle diastolique, de la pression artérielle moyenne, du volume d'éjection et de l'index de résistance périphérique totale.

Ces paramètres hémodynamiques sont obtenus à partir des signaux analogiques mesurés en continu et en temps réel sur les patients, puis numérisés.

Conformément à cet art antérieur, l'analyse spectrale de l'intervalle RR de la PAS, de la pression diastolique, de la pression artérielle moyenne du volume d'éjection systolique et de l'index de résistance périphérique totale, est réalisée en continu et en temps réel. L'inconvénient de cette technique est premièrement qu'elle nécessite des données stationnaires ce qui n'est souvent pas le cas surtout au cours d'une chirurgie sous anesthésie générale. Deuxièmement, cette technique ignore la composante pulsatile du fonctionnement vasculaire.

Les données hémodynamiques mesurées ou calculées sont également utilisées pour le calcul automatique de la sensibilité du baroréflexe du patient. L'algorithme mis en oeuvre recherche les épisodes d'activation spontanée du baroréflexe. Ces épisodes sont définis comme correspondants au cas où l'intervalle RR croît suite à une augmentation de la PAS (séquence +/+) ou l'intervalle RR décroît suite à une diminution de la PAS (séquence -/-), respectivement selon des amplitudes d'au moins 4 millisecondes pour l'intervalle RR et d'au moins un millimètre de mercure pour la PAS, pendant au moins 4 battements cardiaques consécutifs. Les régressions linéaires des augmentations/diminutions de la PAS et des augmentations/diminutions simultanées de l'intervalle RR, sont calculées. L'algorithme fait alors la moyenne des régressions linéaires ainsi obtenues, ce qui donne une pente moyenne (ms/mm Hg) correspondant à la sensibilité du baroréflexe. Un patient dont le système nerveux autonome est affecté, présente une sensibilité du baroréflexe réduite par rapport à un sujet normal.

Cet article ne fait pas allusion à une surveillance en continu et en temps réel de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

L'article de *«Legramente et al., Circulation, 1999, vol.99, 1761-1766»,* montre qu'il existe en matière de régulation nerveuse de la circulation, des séquences non-baroréflexes à rétroaction positive ("feed-forward") comprenant au moins 3 battements, dans lesquels interviennent une diminution de l'intervalle RR simultanément à une augmentation de la PAS (-/+). Selon les auteurs de cet article, ces séquences non-baroréflexes sont l'expression d'une régulation cardiovasculaire à court terme à rétroaction positive (feed-forward) intégré et sous influence nerveuse. Cette régulation est capable d'interagir dynamiquement avec les mécanismes à rétroaction négative ("feed-back") d'origine baroréflexe, dans le contrôle de la fréquence cardiaque. Ces séquences non-baroréflexes sont sous influence des systèmes nerveux sympathique et parasympathique.

L'article de Legramente et al. ne fournit aucune indication quant à l'utilisation de séquences non-baroréflexes RR(-) / PAS(+) comme signal prédictif en continu et en temps réel d'événements anormaux pour des patients sous surveillance accrue (anesthésie, soins intensifs, etc.) à cet égard. Au contraire, Legramente émet l'hypothèse que dans des conditions normales, les mécanismes neuronaux responsables d'une régulation à rétroaction positive ("feed-forward") pourraient être en opposition permanente avec les mécanismes du baroréflexe responsables d'une régulation à rétroaction négative ("feed-back"). En outre, Legramente ne divulgue pas le filtrage des signaux RR et PAS.

La demande PCT WO2004034897 concerne un procédé et un appareil permettant de surveiller une réaction d'un patient sous anesthésie ou sous sédation. Pour cela, un capteur est présent, lequel permet d'acquérir un signal qui représente un écoulement cardiovasculaire périphérique chez le patient. Des pulsations cardiaques successives sont détectées et des paramètres d'ondes pulsatiles prédéterminés sont mesurés de manière répétée dans ledit signal, des paramètres d'ondes pulsatiles prédéfinis étant également mesurés dans lesdites pulsations cardiaques. Tout d'abord, lesdits paramètres d'ondes pulsatiles prédéterminés dans un certain nombre de pulsations cardiaques successives sont comparés à une apparition possible d'une modification substantielle. Ensuite, si la modification est notée, une valeur de référence statistique est calculée par dérivation de valeurs à partir desdits paramètres d'ondes pulsatiles prédéfinis, et la/les modification(s) entre au moins un paramètre d'ondes pulsatiles prédéfini et ladite valeur de référence est/sont quantifiées. Les résultats et/ ou résultats intermédiaires sont enfin affichés et/ou enregistrés.

Le brevet US-B-5,437,285 décrit un procédé et un dispositif de prédiction de mort cardiaque subite, par évaluation simultanée de l'influence du système nerveux autonome sur le coeur et de la stabilité électrique cardiaque.

VERRIER et NEARING, les inventeurs cités dans ce brevet, se sont fixés pour objectif de mettre au point une méthode dynamique non invasive d'évaluation de la vulnérabilité de patients vis-à-vis de la fibrillation ventriculaire.

Pour cela, l'alternance de l'onde T, la variabilité du rythme cardiaque et l'ampleur de la dispersion de l'intervalle QT sont évaluées simultanément. Cette évaluation se fait battement-par-battement, dans les intervalles RR successifs. L'algorithme utilisé crée donc une suite d'intervalles RR pour former un signal RR.

La variabilité du rythme cardiaque est estimée en calculant les composantes de basse fréquence autour de 0.1 Hz (low frequency: LF), les composantes de haute fréquence autours de 0.35Hz (high frequency: HF) ainsi que le rapport LF/HF dans le spectre de la fréquence cardiaque calculée battement-après-battement.

Le procédé selon ce brevet propose également la mesure de la sensibilité du baroréflexe. Ce paramètre est obtenu à partir de la pression artérielle, de la fréquence cardiaque instantanée et d'un signal représentant le volume pulmonaire instantané, à l'aide d'une technique fondée sur un modèle auto-régressif à moyenne glissante (Autoregressive Moving Average: ARMA).

Ce brevet ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet US-B-5,419,338 concerne un procédé et un dispositif de détection de déséquilibres dans le contrôle du système cardiovasculaire par le système nerveux autonome parasympathique/sympathique. Il s'agit d'une méthode directe d'évaluation des déséquilibres des contrôles sympathique/parasympathique et d'indication des prédispositions à la mort cardiaque subite. Selon ces documents, on analyse la variabilité de l'intervalle QT simultanément à la variabilité de l'intervalle RR, et on procède à l'analyse spectrale des signaux RR et QT. L'indicateur des déséquilibres du système nerveux doit être autonome et donné par l'analyse des densités de fréquence QT versus RR. Il est clair que ce document ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet américain US-B-5,967,995 décrit un système pour la prédiction des arythmies cardiaques à risque. Selon ce procédé, le signal RR est décomposé et transformé en coefficients de transformation KARHUNEN LOEVE. Ce sont ces coefficients qui sont utilisés comme indices prédictifs d'accidents cardiaques. Ce procédé fait intervenir l'analyse spectrale du signal RR.

Ce brevet américain ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

La demande PCT WO-A-95/03739 divulgue un procédé de mesure de l'activité du système nerveux autonome, selon lequel on mesure en continu une suite d'intervalles RR à partir d'un ECG et on réalise une représentation de Poincaré à partir des intervalles RR en continu et en temps réel. Le niveau de l'activité sympathique du patient peut être quantifié en déterminant la dimension de corrélation correspondant à la représentation graphique de Poincaré. Le niveau de désordre cardiaque est apprécié en constatant dans quelle mesure la dimension de corrélation est hors d'un intervalle prédéterminé. Le niveau d'activité parasympathique peut également être quantifié à partir de la largeur de l'ensemble de points de la représentation de Poincaré. Cet enseignement technique antérieur ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet américain US-B-5,439,004 concerne un système et un procédé de détection de fibrillations ventriculaires fondé sur la théorie du chaos. Selon ce procédé, on a recours à une représentation de Poincaré de l'amplitude du signal ECG. Cet enseignement technique antérieur ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet français FR-B- 2747027 divulgue un procédé et un dispositif de détermination de la profondeur d'anesthésie d'un patient auquel au moins un produit anesthésiant est administré, comportant une étape (10) d'acquisition d'au moins un signal représentatif de l'activité du coeur du patient, une étape (12) de détection de la position d'une onde périodique déterminée dans chaque signal du coeur du patient, une étape (13) de calcul d'intervalles temporels entre lesdites ondes périodiques, une étape (14) de détermination de séries numériques d'intervalles temporels, une étape (15) de calcul d'une dimension fractale desdites séries d'intervalles temporels et une étape (16) de calcul de la profondeur d'anesthésie en fonction de la dimension fractale.

L'étape (10) d'acquisition d'au moins un signal consiste à mesurer au moins un -voire au moins deux- des signaux suivants du patient: l'électrocardiogramme, le débit sanguin, l'absorption lumineuse par le sang, la pression artérielle, la teneur en oxygène du sang ou bien encore un signal acoustique émis par le coeur. L'étape (15) de calcul d'une dimension fractale desdites séries d'intervalles temporels consiste à calculer une dimension de corrélation de ces séries. L'étape (16) de calcul de la profondeur d'anesthésie en fonction de la dimension fractale consiste à déterminer la dimension fractale de séries numériques d'intervalles temporels avant l'administration d'au moins un produit anesthésiant au patient, à définir un coefficient de normalisation tel que le produit de ce coefficient par la dimension fractale soit sensiblement égal à une valeur de référence et à multiplier la dimension fractale de séries d'intervalles temporels après l'administration d'au moins un produit anesthésiant au patient, par le coefficient de normalisation.

Cet enseignement technique antérieur ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet américain US-5,372,140 divulgue une méthode et un appareil pour mesurer la profondeur de l'anesthésie en temps réel en mesurant l'arythmie sinusale. Selon cette méthode, les suites des intervalles RR sont analysées pour déterminer la position dans le temps de chaque onde R par rapport au cycle respiratoire. Lorsque le cycle respiratoire est représenté sous forme d'un cercle, à chaque onde R on associe une position sur ce cercle ainsi qu'un vecteur dont l'origine est dans le centre du cercle est qui est orienté vers la position de l'onde R sur le cercle. Un vecteur résultant est calculé et puis comparé à un vecteur de référence pour dériver un index de la profondeur de l'anesthésie. Le vecteur de référence est calculé à l'aide du test Reyleigh en fonction d'un niveau de probabilité prédéfini qui peut être sélectionné par l'utilisateur. La méthode utilise comme signaux d'entrée l'électrocardiogramme et un signal respiratoire. Cet enseignement technique antérieur ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Le brevet américain US-6,685,649 divulgue une méthode de monitorage de l'état du patient sous anesthésie ou sédation. Selon cette méthode, un signal (S) est acquis, représentant l'activité cardiovasculaire du patient. Le signal (S) peut être préférentiellement l'électrocardiogramme, mais aussi la pression artérielle, le taux d'oxygène dans le sang, etc. Des ondes répétitives (P) sont détectées dans le signal (S) et le calcul des intervalles temporels (Ti) ou des pressions (Bi) ou des fréquences temporelles (Ri) est réalisé à partir des ces ondes successives. Les suites temporelles des intervalles (Ti), ou des pressions (Bi) ou des fréquences (Ri) sont filtrées donnant naissance à des suites moyennées. Ce filtrage élimine les variations rapides contrôlées par le système parasympathique. Des activations présumées du système sympathique sont ensuite recherchées dans les suites moyennées en détectant des diminutions substantielles des intervalles temporels (par ex. intervalles RR) ou de manière alternative des augmentations substantielles de la fréquence cardiaque ou des augmentations substantielles de la pression artérielle (par ex. pression artérielle systolique). La détection des diminutions, respectivement les augmentations des signaux moyennées est réalisée par le calcul de la dérivée des suites moyennées. Dans la suite des dérivées des suites moyennées, les dérivées correspondant à des activations cardiovasculaires sympathiques sont accentuées tandis que les autres dérivées sont supprimées ou négligées à l'aide d'un opérateur mathématique. Dans les suites des dérivées ainsi sélectionnées, nommées accélérations, une moyenne glissante est appliquée en donnant naissance à un index d'accélération (par ex. index d'accélération de la fréquence cardiaque). L'index d'accélération est selon l'inventeur un index de l'adéquation de l'analgésie pour des patients sous anesthésie générale ou sédation. L'index de l'accélération de la fréquence cardiaque a été récemment couplé avec l'index de l'entropie de l'électromyogramme frontal (Rantanen, M. et al. Anesthesiology, 2004, vol. 101: A559). Cet enseignement technique antérieur ne fait pas allusion à une surveillance en continu et en temps réel, de la survenue concomitante d'une inefficacité temporaire du baroréflexe cardiaque et d'une activation de la régulation cardiovasculaire non-baroréflexe, comme témoin prédictif d'événements médicaux (par ex. réveil d'anesthésie), nécessitant une parade médicale idoine.

Des méthodes fondées sur l'électroencéphalogramme (EEG) ou les potentiels évoqués auditifs ont été développés récemment et mesurent la composante hypnotique de l'anesthésie. Les index dérivés de l'EEG reflètent l'activité du cortex cérébral. L'index le plus connu dérivé de l'EEG est l'index bispectral (Bispectral Index: BIS®, Acpect, Natick MA) dont l'idée initiale a été divulguée par le brevet américain US-4,907,597. D'autres index existent également fondés sur l'EEG, tels a) le front de fréquence spectral (Spectral Edge Frequency: SEF) ou la fréquence médiane (Median Frequency: MF) à partir du spectre EEG, b) l'index de l'état du patient (Patient State Index: PSI, Physiometrix, N. Billerica, Mass), c) l'entropie de l'EEG (M-Entropy index, S/5 Entropy Module, Datex-Ohmeda, Helsinki, Finlande), d) l'index Narcotrend (Hambourg, Allemagne), e) la complexité Lempel-Ziv de l'EEG, etc.

Les potentiels évoqués auditifs sont mesurés à l'aide de l'index AAI (A-line ARX Index) calculé en temps réel par le moniteur A-line® (Danmeter, Odense, Danemark). L'index AAI reflète un mélange de l'activité corticale et sous-corticale. Toutefois, l'index AAI est aussi peu performant que l'index BIS pour prédire le mouvement intra-opératoire en réponse à un stimulus nociceptif (Struys, M. M. et al. Anesthesiology, 2002, vol. 96: 803-816): la réponse motrice à un stimulus nociceptif pourrait être l'effet d'un réflexe spinal et non pas supraspinal. Au total, les moniteurs fondés sur l'EEG spontané ou des potentiels évoqués ne sont pas performants dans l'évaluation de l'analgésie, leur index augmentant souvent en même temps ou même après la survenue du mouvement intra-opératoire.

Objectifs de l'invention

Pour pallier la carence de l'art antérieur, l'un des objectifs essentiels de l'invention est de fournir un procédé et un dispositif performants et économiques et simples, permettant prédire de manière fiable et certaine la survenue d'événements médicaux imprévus (par ex. mouvement intra-opératoire inopiné en réponse à une stimulation nociceptive, réveil d'anesthésie, allègement de l'anesthésie générale, notamment de la composante analgésique, etc.), nécessitant une parade médicale idoine.

Un autre objectif essentiel de l'invention est de fournir un procédé et un dispositif de monitorage simple, fiable, performant, économique et adéquat de l'anesthésie de façon à permettre un déroulement optimisé des actes chirurgicaux réalisés sous anesthésie générale, et ce sans problème de sous-dosage ou surdosage d'anesthésiques.

Un autre objectif essentiel de l'invention est de fournir un procédé et un dispositif fiables, performants et économiques, notamment :

o d'aide au diagnostic en temps réel en soins intensifs de cardiologie ou en cardiologie ambulatoire (prévision de l'ischémie myocardique ou de la fibrillation ventriculaire),
o de monitorage de l'anesthésie pour d'autres facteurs que la profondeur d'anesthésie et la prédiction du réveil (ischémie myocardique postopératoire, anesthésie locorégionale, douleur, etc...),
o de réanimation médicale ou chirurgicale (adulte, pédiatrique, néonatalogie),
o d'obstétrique,
o de médecine d'urgence/accueil, oxyologie,
o de médecine spatiale,
o d'exploration fonctionnelle du système nerveux autonome (syncope, diabète, dystonie neurovégétative...).

Description de l'invention

Ces objectifs, parmi d'autres ont été atteints par l'invention qui concerne tout d'abord un procédé de prédiction d'évènements médicaux anormaux et/ou d'aide au diagnostic et/ou de monitorage caractérisé en ce qu'il consiste essentiellement à détecter, en continu et en temps réel, de la survenue concomitante d'une inefficacité temporaire du baroréflexe cardiaque et d'une activation de la régulation cardiovasculaire non-baroréflexe. L'invention vise également un dispositif, en particulier pour la mise en oeuvre du susdit procédé, caractérisé en ce qu'il comprend des moyens de détection, en continu et en temps réel, de la survenue concomitante d'une inefficacité temporaire du baroréflexe cardiaque et d'une activation de la régulation cardiovasculaire non-baroréflexe.

Les inventeurs ont eu le mérite, après de longues et laborieuses recherches de montrer qu'il est possible d'utiliser toute survenue concomitante d'une inefficacité temporaire du baroréflexe cardiaque et d'une activation de la régulation cardiovasculaire non-baroréflexe, comme témoin prédictif d'événements médicaux anormaux. En d'autres termes, on assimile, selon l'invention, la survenue concomitante d'une inefficacité temporaire du baroréflexe cardiaque et d'une activation de la régulation cardiovasculaire non-baroréflexe, par exemple, à un index d'allègement de la composante analgésique de l'anesthésie et de prédiction du mouvement intra-opératoire inopiné.

Plus précisément, l'invention a consisté à sélectionner des signaux cardiovasculaires instantanés particuliers, pour mettre en évidence une telle survenue concomitante d'une inefficacité temporaire du baroréflexe cardiaque et d'une activation de la régulation cardiovasculaire non-baroréflexe.

Ainsi, le procédé selon l'invention comprend les étapes suivantes :

a. mesure en continu (battement-après-battement) des intervalles temporels entre 2 cycles cardiaques consécutifs (IT) et de la pression artérielle (PA);
b. filtrage à l'aide d'un filtre passe-bas des suites battement-après-battement des intervalles temporels IT et des suites de PA calculées à l'étape a), pour éliminer les variations rapides sous contrôle parasympathique, à la fréquence ventilatoire supérieure ou égale à un seuil compris entre 0,1 et 0.15 Hz, ce filtrage donnant naissance à des suites d'IT et de PA filtrées $IT_f$, respectivement $PA_f$.
c. surveillance de la survenue concomitante d'une inefficacité temporaire du baroréflexe cardiaque et d'une activation de la régulation cardiovasculaire non-baroréflexe au travers de la manifestation d'événements choisis dans le groupe de séquences suivantes:

o augmentation de $PA_f$ / délai / augmentation réduite de $IT_f$;
o augmentation de $PA_f$/ délai / augmentation retardée de $IT_f$;

o augmentation de $PA_f$/ délai / diminution de $IT_f$;

o ainsi que la combinaison d'au moins deux de ces séquences;

d. émission d'une alarme pour avertir de la survenue concomitante d'une inefficacité temporaire du baroréflexe cardiaque et d'une activation de la régulation cardiovasculaire non-baroréflexe selon l'étape c).

Le dispositif selon l'invention comprend essentiellement :

(a) des moyens de mesure en continu (battement-après-battement) des intervalles temporels entre 2 cycles cardiaques consécutifs (IT) et de la pression artérielle (PA);

(b) un filtre passe-bas pour filtrer des suites battement-après-battement des intervalles temporels IT et des suites de PA précédemment mesurées, pour éliminer les variations rapides sous contrôle parasympathique, à la fréquence ventilatoire supérieure ou égale à un seuil compris entre 0,1 et 0.15 Hz, ce filtrage donnant naissance à des suites d'IT et de PA filtrées $IT_f$, respectivement $PA_f$;

(c) des moyens de surveillance de la survenue concomitante d'une inefficacité temporaire du baroréflexe cardiaque et d'une activation de la régulation cardiovasculaire non-baroréflexe au travers de la manifestation d'événements choisis dans le groupe de séquences suivantes :

■ augmentation de $PA_f$ / délai / augmentation réduite de $IT_f$;

■ augmentation de $PA_f$ / délai / augmentation retardée de $IT_f$;

■ augmentation de $PA_f$/ délai / diminution de $IT_f$;

■ ainsi que la combinaison d'au moins deux de ces séquences ;

(d) et au moins une alarme pour avertir de la survenue concomitante d'une inefficacité temporaire du baroréflexe cardiaque et d'une activation de la régulation cardiovasculaire non-baroréflexe.

Les intervalles temporels IT sont, de préférence, calculés soit à partir des intervalles RR, c'est-à-dire des intervalles de temps entre deux complexes QRS de l'ECG, soit à partir des intervalles entre deux points caractéristiques (débuts de montée ou maximums ou autres) de la PA en continu ou de la saturation en oxygène du sang (Sp02) en continu. Avantageusement, le dispositif de l'invention comprend des moyens pour calculer les intervalles IT comme expliqué dans le paragraphe précédent.

La PA est choisie dans le groupe de signaux comprenant: la pression artérielle systolique (PAS) du patient, la pression artérielle diastolique (PAD) du patient, la pression artérielle moyenne (PAM) du patient, la PAS étant préférée.

La PA en continu est, de préférence, obtenue soit par mesure directe invasive ou non-invasive, soit par mesure indirecte de préférence à partir de la SpO2 en continu, calibrée à l'aide des valeurs intermittentes de PA obtenues de préférence à l'aide d'un brassard à tension.

Avantageusement, le dispositif de l'invention comprend des moyens de mesure de la PA en continu, ces moyens fonctionnant comme cela est expliqué au paragraphe précédent.

Dans le cas où les suites de PA en continu sont obtenues de manière indirecte à l'aide de la SpO2 en continu calibrée avec les valeurs intermittentes de PA, le procédé selon l'invention comprend, de préférence, les étapes suivantes:

a1) la suite des maximums du signal SpO2 lors de chaque cycle cardiaque est calculée;

a2) la suite obtenue à l'étape a1) est inversée par soustraction d'une constante strictement supérieure à l'amplitude maximale du signal SP02;

a3) calibrage de la suite obtenue à l'étape a2) en unités de pression en appliquent un opérateur linéaire du $1^{er}$ degré à la suite obtenue à l'étape a2), les coefficients de cet opérateur étant obtenus à partir des valeurs intermittentes de PA.

Le dispositif de l'invention comprend avantageusement les moyens de calcul, les moyens de vision et les moyens de calibrage de la suite, pour permettre la mise en oeuvre des étapes, respectivement a1), a2) et a3) sus évoquées.

Les suites IT et PAS présentent des variations continuelles d'au moins 3 types:

a) liées à la ventilation (> 0.1 Hz), éliminées selon l'invention par un filtre,

b) autour de 0.1Hz (ondes de Mayer) et

c) plus lentes.

Les variations rapides liées à la ventilation sont sous contrôle parasympathique. En les éliminant lors du filtrage passe-bas, seules les variations sous contrôle sympathique restent à analyser.

Le filtrage passe-bas des suites d'IT et de PA est, de préférence, réalisé en temps réel à l'aide d'au moins un filtre à réponse impulsionnelle infinie (RII) ou à réponse impulsionnelle finie, ou à l'aide de tout autre type de filtre passe-bas performant, le filtre de type RII étant préféré.

Le dispositif de l'invention comprend avantageusement au moins un filtre tel que défini dans le paragraphe précédent.

Pour une information rapide et claire des praticiens, il est prévu, de préférence, selon l'invention qu'au moins l'un des signaux suivants soit affiché sur au moins un écran: les suites d'IT et de PA obtenues selon le point a) et les suites filtrées d'$IT_f$ et $PA_f$ obtenues selon le point b).

Le dispositif de l'invention comprend avantageusement au moins un écran permettant l'affichage de ce qui est défini dans le paragraphe précédent

Normalement, le baroréflexe cardiaque, en vertu d'une régulation à rétroaction négative (feed-back), fait correspondre à des montées des PAS, après un délai variable (1-20s), une bradycardie c'est-à-dire des augmentations des intervalles temporels IT. Généralement, lors d'un allègement de l'anesthésie (notamment de la composante analgésique) le patient commence à percevoir une douleur suite à des stimulations nociceptives, d'abord au niveau du subconscient, puis de manière ultime au niveau du conscient à l'émergence complète. Les voies afférentes de la nociception projettent sur des zones sous-corticales comme l'hypothalamus ou la matière grise péri-aquéductale. Une activation des ces zones sous-corticales se traduit par une tachycardie et une hypertension induites par des afférences non-cardiovasculaires qui viennent occlure l'arc baroréflexe. Par conséquent, la bradycardie induite par l'arc baroréflexe suite à une montée de pression est en concurrence permanente avec une possible tachycardie non-baroréflexe induite par les zones sous-corticales susmentionnées suite à la stimulation nociceptive. Un équilibre baroréflexe/non-baroréflexe existe à chaque instant.

Lors d'un allègement de l'anesthésie (notamment de la composante analgésique), des épisodes de courte durée existent pendant lesquels le fonctionnement du baroréflexe est occlut par un fonctionnement non-baroréflexe selon ce qui suit: les montées des PAS sont suivies soit par une très faible montée de l'IT (baroréflexe peu efficace), soit par une montée retardée de l'IT (baroréflexe retardé), soit par une diminution de l'IT (tachycardie signant un baroréflexe non-fonctionnel, occlu par une régulation cardiovasculaire non-baroréflexe), à l'inverse du fonctionnement attendu du baroréflexe cardiaque. Cet allègement de l'anesthésie est généralement à l'origine du mouvement intra-opératoire inopiné suite à une stimulation nociceptive.

L'un des points forts de l'invention est notamment d'avoir isolé et quantifié cette survenue concomitante d'une inefficacité temporaire du baroréflexe cardiaque et d'une activation de la régulation cardiovasculaire non-baroréflexe, en temps réel à l'aide d'un algorithme capable de déclencher une alarme.

La survenue concomitante d'une inefficacité temporaire du baroréflexe cardiaque et d'une activation de la régulation cardiovasculaire non-baroréflexe, est effectuée, de préférence, à l'aide d'au moins quatre paramètres suivants:

p1) le rapport entre l'aire sous la courbe d'une séquence d'$IT_f$ dite réponse baroréflexe d'$IT_f$ et l'aire sous la courbe de la montée de $PA_f$ qui a provoqué ladite réponse baroréflexe d'$IT_f$,
p2) l'amplitude de la baisse de l'$IT_f$ qui a précédé la montée d'$IT_f$ qui suit ladite montée de $PA_f$,
p3) le rapport entre l'amplitude de ladite réponse baroréflexe d'$IT_f$ et l'amplitude de ladite baisse d'$IT_f$,
p4) la différence algébrique $\Delta IT$ entre la valeur d'$IT_f$ après une période de temps dite d'estimation à partir du début de ladite réponse baroréflexe d'$IT_f$, et la valeur d'$IT_f$ au début de ladite réponse baroréflexe d'$IT_f$ .

ainsi que, toute combinaison pondérée ou pas des paramètres précédents.

Dans le dispositif de l'invention, les moyens (c) de surveillance mettent avantageusement en oeuvre au moins les quatre paramètres p1), p2), p3) et p4) définis au paragraphe précédent.

Dans le procédé ou le dispositif selon l'invention, la réponse baroréflexe d'$IT_f$ provoquée par une montée de $PA_f$, est, de préférence :

● soit la montée d'$IT_f$ qui suit ladite montée de $PA_f$, si ladite montée d'$IT_f$ commence dans un intervalle de temps compris entre une limite inférieure égale à 0s et une limite supérieure comprise entre 10s et 20s par rapport au début de ladite montée de $PA_f$;
● soit la séquence d'$IT_f$ qui commence après un intervalle compris entre 5s et 15s par rapport au début de ladite montée de $PA_f$, dans le cas où la montée d'$IT_f$ qui suit ladite montée de $PA_f$, commence après ladite limite supérieure comprise entre 10s et 20s par rapport au début de ladite montée de $PA_f$;

dans les deux cas, la durée de la réponse baroréflexe d'$IT_f$ étant égale à ladite période d'estimation ; l'amplitude de ladite réponse baroréflexe d'$IT_f$ étant la différence entre le maximum atteint par l'$IT_f$ au cours de sa montée pendant la durée de ladite réponse baroréflexe d'$IT_f$, et le niveau d'$IT_f$ au début de la montée d'$IT_f$ qui suit ladite montée de $PA_f$.

Dans le procédé ou le dispositif selon l'invention, les montées de $PA_f$ prises en considération, sont de préférence celles dont l'amplitude est supérieure à un seuil de pression compris entre 1mmHg et 5mmHg, de préférence entre 2mmHg et 4 mmHg, et mieux encore entre 2 mmHg et 3 mmHg, les autres montées de $PA_f$ étant négligées.

Dans le procédé ou le dispositif selon l'invention, la période dite d'estimation est de préférence égale à la durée de ladite montée de $PA_f$ multipliée par un coefficient compris entre 0.5 et 2, de préférence entre 1 et 1.8.

De préférence, lesdites aires sous les courbes de la réponse baroréflexe d'$IT_f$ et de la montée de $PA_f$ sont calculées entre un moment dit de référence et pendant ladite période d'estimation en réalisant pour chaque échantillon de ladite courbe au cours de ladite période d'estimation à partir dudit moment de référence, la somme des différences algébriques entre la valeur de l'échantillon à un moment donné et la valeur de la courbe audit moment de référence. Etant des sommes algébriques, lesdites aires peuvent être aussi bien positives que négatives.

Le dispositif selon l'invention comprend avantageusement des moyens de calcul des susdites aires, ces moyens fonctionnent comme expliqué au paragraphe précédent.

D'autres paramètres cardiovasculaires connus et applicables aux signaux IT ou PA en fonction du temps peuvent être utilisés en combinaison avec les paramètres p1-p4, tels le rapport entre les puissances de basse et haute fréquence (LF/HF), coefficient alpha, cohérence, analyses à partir de la représentation de Poincaré, dimension fractale, pente bêta, analyse non-linéaires, analyse temps-fréquence, analyse par ondelettes, témoignant d'une inefficacité du baroréflexe et/ou d'une activation sympathique et/ou d'une inhibition parasympathique sous le contrôle des zones sous-corticales stimulées par les récepteurs nociceptifs.

De préférence, au moins une alarme (d) est destinée à prédire tout allègement programmé ou intempestif de l'anesthésie, notamment de la composante analgésique. Cette alarme destinée à avertir de la survenue concomitante d'une inefficacité temporaire du baroréflexe cardiaque et d'une activation de la régulation cardiovasculaire non-baroréflexe, selon l'étape d), est donnée par affichage, en continu et en temps réel, d'un index prédictif exprimant la profondeur cardiovasculaire de l'anesthésie (CARDEAN©: Cardiovascular Depth of Anesthesia).

Le dispositif selon l'invention comprend avantageusement au moins une alarme et des moyens d'affichage de cette alarme, comme expliqué au paragraphe précédent.

L'index de la profondeur de l'anesthésie selon l'invention est utilisable, de préférence uniquement à partir du moment où le patient est déjà anesthésié et tous les signes de Guedel sont inhibés, à savoir le réflexe ciliaire, le réflexe cornéen, le larmoiement, le mouvement oculaire et la réponse motrice à la stimulation nociceptive.

L'étape c) de la surveillance de la survenue concomitante d'une inefficacité temporaire du baroréflexe cardiaque et d'une activation de la régulation cardiovasculaire non-baroréflexe, ainsi que l'émission d'une alarme selon l'étape d), sont régies, de préférence, de manière alternative par au moins l'un des algorithmes suivants :

A1) l'index prédictif exprimant la profondeur cardiovasculaire de l'anesthésie indique un allègement de l'anesthésie, notamment de la composante analgésique, et émet une alarme, lorsqu'au moins les quatre conditions suivantes sont respectées:

● le paramètre p1 est au-dessous d'un seuil s1 compris entre 0 et 4, de préférence entre 1 et 3,
● le paramètre p2 est au-dessus d'un seuil s2 compris entre 30ms et 300ms, de préférence entre 40ms et 80ms,
● le paramètre p3 est au-dessous d'un seuil s3 compris entre 0 et 1, de préférence entre 0.25 et 0.75, de préférence entre 0.3 et 0.75,
● le paramètre p4 est au-dessous d'un seuil s4 compris entre -100 et 25, de préférence entre -40 et 10, de préférence entre -20 et 10.

ou,

A2) l'index prédictif exprimant la profondeur cardiovasculaire de l'anesthésie et indiquant un allègement de l'anesthésie, notamment de la composante analgésique, et émettant une alarme, est obtenu en utilisant un réseau des neurones ayant comme paramètres d'entrée les paramètres de p1 à p4, et ayant des conditions d'apprentissage de manière obtenir une combinaison optimale de sensibilité et spécificité, à l'aide d'une courbe ROC (Receiver Operator Characteristic curve).

Avantageusement, les moyens (c) de surveillance du dispositif selon l'invention et l'alarme (d) sont régis par A1) ou A2), comme cela est défini ci-dessus.

Selon un autre de ses aspects l'invention concerne un programme d'ordinateur, en particulier pour la mise en oeuvre du procédé tel que défini supra ou pour régir les moyens (c) et l'alarme (d) du dispositif tel que défini supra. Ce procédé est caractérisé en ce qu'il comprend des moyens de code de programme pour effectuer la totalité des étapes de(s) l'algorithme(s) A1) et/ou A2) tel(s) que défini(s) ci-dessus, lorsque ledit programme fonctionne sur un ordinateur.

Un autre objet de l'invention vise un produit de programme d'ordinateur, en particulier pour la mise en oeuvre du procédé tel que défini supra, caractérisé en ce qu'il comprend des moyens de code de programme, stockés sur un milieu lisible par un ordinateur, pour effectuer la totalité des étapes de(s) l'algorithme(s) A1) et/ou A2) tel(s) que défini(s) ci-dessus, lorsque ledit produit de programme fonctionne sur un ordinateur.

Le logiciel selon l'invention pourrait être inclus seul ou en combinaison avec d'autres méthodes de surveillance (EEG, potentiels évoqués, etc.) dans une boucle de rétroaction pour ajuster l'administration de médicaments en anesthésie, soins intensifs, ou plus généralement en médecine.

L'invention est également relative à un dispositif, en particulier pour la mise en oeuvre du procédé selon l'invention et tel que défini ci-dessus. Ce dispositif est caractérisé en ce qu'il comprend essentiellement :

a) des moyens de mesure en continu (battement-après-battement) des intervalles temporels entre 2 cycles cardiaques consécutifs (IT) et de la pression artérielle (PA) ;
b) des moyens de filtrage à l'aide d'un filtre passe-bas des suites battement-après-battement des intervalles temporels IT et des suites de PA calculées à l'étape a), pour éliminer les variations rapides sous contrôle parasympathique, à la fréquence ventilatoire supérieure ou égale à un seuil compris entre 0,1 et 0.15 Hz, ce filtrage donnant naissance à des suites d'IT et de PA filtrées $IT_f$, respectivement $PA_f$;
c) des moyens de surveillance de la survenue concomitante d'une inefficacité temporaire du baroréflexe cardiaque et d'une activation de la régulation cardiovasculaire non-baroréflexe au travers de la manifestation d'événements choisis dans le groupe de séquences suivantes:

■ augmentation de $PA_f$ / délai / augmentation réduite de $IT_f$;
■ augmentation de $PA_f$ / délai / augmentation retardée de $IT_f$;
■ augmentation de $PA_f$ / délai / diminution de $IT_f$;
■ ainsi que la combinaison d'au moins deux de ces séquences;

d) au moins une alarme pour avertir de la survenue concomitante d'une inefficacité temporaire du baroréflexe cardiaque et d'une activation de la régulation cardiovasculaire non-baroréflexe selon l'étape c).

Ces moyens constitutifs du dispositif sont décrits plus en détail ci-dessous, ainsi que dans les exemples ci-après.

Le dispositif selon l'invention peut être utilisé sous forme:

i) d'un module supplémentaire qui peut se glisser dans les moniteurs modulaires existants (type HP®/Philips® ou Datex®);
ii) d'un insert logiciel lors d'une simple mise à niveau logiciel.
Dans les cas a) et b), ce dispositif utiliserait les signaux ECG et/ou Sp02 couplés avec les valeurs intermittentes de PA disponibles dans toutes les salles d'opération;
iii) d'un module indépendant intégré par exemple dans un moniteur de PA continue non-invasive, (dans ce cas les suites IT seront les intervalles de temps entre deux points caractéristiques de la PA).

Ce dispositif peut également servir chez l'animal ou dans les domaines suivants, aussi bien en intra-opératoire qu'à l'extérieur de la salle d'opération: aide au diagnostic en temps réel en soins intensifs de cardiologie ou en cardiologie ambulatoire (prévision de l'ischémie myocardique, prévision de fibrillation ventriculaire), anesthésie (ischémie myocardique postopératoire, anesthésie locorégionale, douleur, etc...), réanimation médicale ou chirurgicale (adulte, pédiatrique, néonatalogie), obstétrique, médecine d'urgence/accueil, oxyologie, médecine spatiale, exploration fonctionnelle du système nerveux autonome (syncope, diabète, dystonie neurovégétative...).

Exemple. Essai clinique

1/ Matériel et méthodes

Un patient (ASA-I, 33 ans) a subi une intervention de ligamentoplastie du genou sous anesthésie générale réalisée avec propofol (DIPRIVAN®) et rémifentanil (ULTIVA®). Les signaux cardiovasculaires suivants ont été enregistrés en continu tout au long de l'intervention chirurgicale: l'électrocardiogramme (ECG) et la pression artérielle non-invasive (FINAPRES 2300, Ohmeda, Englewood, CO). Des index de la profondeur de l'anesthésie dérivés de l'électroencéphalogramme (EEG) ont été également enregistrés en continu tout au long de l'intervention chirurgicale: l'index BIS® (Aspect, Natick, MA, USA), version 2002, et l'index AAI (A-Line®, Danmeter A/S, Odense, Danemark). L'acquisition des signaux cardiovasculaires et dérivés de l'EEG a été effectuée à l'aide d'un ordinateur portable: via une carte d'acquisition (KPCMCIA 16AIAO, Keithley, Cleveland, Ohio) pour les signaux cardiovasculaires et via les ports série RS232 pour les signaux dérivés de l'EEG. L'acquisition, le stockage et le traitement des données ont été réalisées à l'aide du logiciel RECAN© (Alpha-2, Lyon, France).

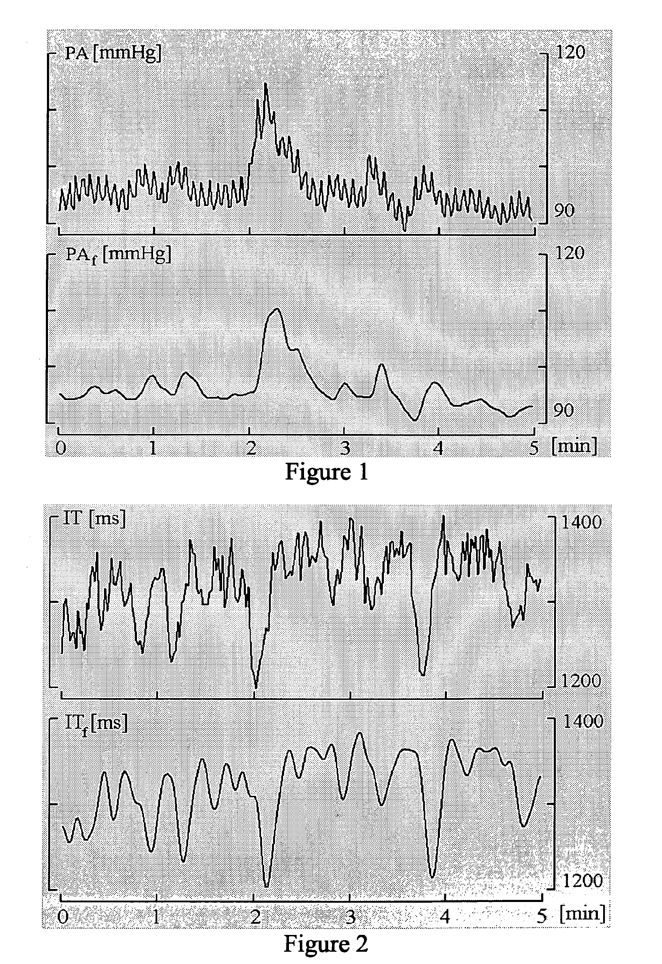

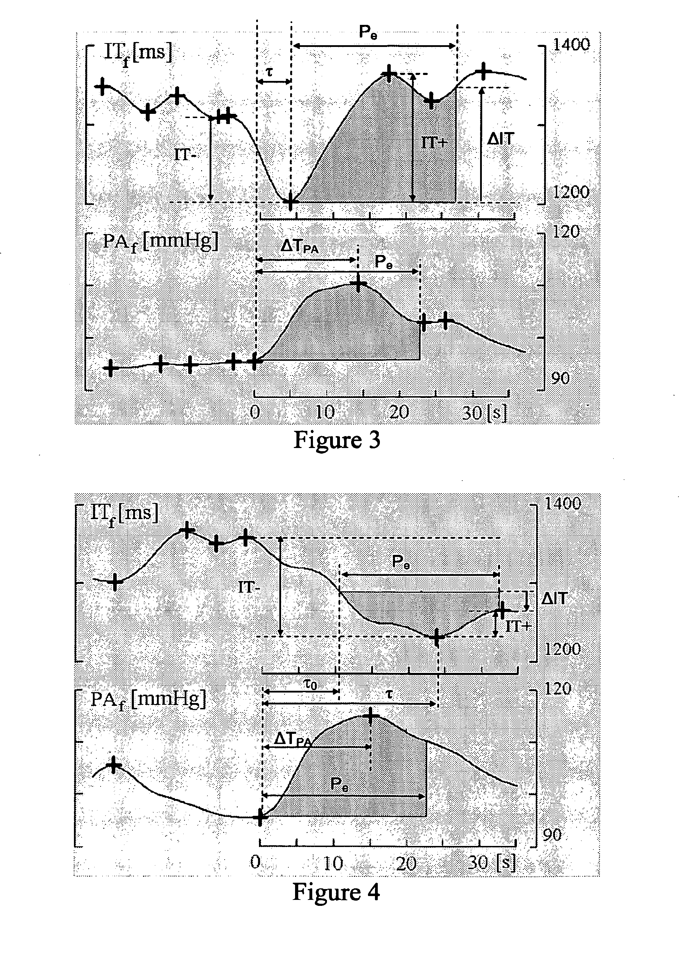

Les suites d'intervalles temporels (IT) entre 2 cycles cardiaques ont été obtenues battement-après-battement en calculant les intervalles de temps (intervalles R-R) entre 2 complexes QRS de l'ECG. Les suites des pressions artérielles (PA) ont été obtenues battement-après-battement en calculant les pressions artérielles systoliques pour chaque cycle cardiaque. Les suites d'IT et de PA ont été filtrées à l'aide d'un filtre passe-bas à réponse impulsionnelle infinie, ici de préférence un filtre de Butterworth dont la fréquence de coupure a été comprise entre 0.1Hz et 0.15Hz. Ce filtrage a donné naissance à des suites filtrées $IT_f$ et $PA_f$ dans lesquelles les variations ventilatoires sous contrôle parasympathique ont été supprimées. Le filtrage des variations ventilatoires (ici à 0.22Hz) est illustré dans les figures 1 et 2. Les minimum et les maximum locaux ont été détectés dans les signaux $IT_f$ et $PA_f$ et sont illustrés par des croix dans les figures 3 et 4. L'amplitude de chaque montée de $PA_f$ a été calculée en effectuant la différence entre chaque maximum local de $PA_f$ et le minimum local de $PA_f$ précédent. Pour chaque montée de $PA_f$ dont l'amplitude a été supérieure à un certain seuil compris entre 1mmHg et 5mmHg, une procédure de calcul de l'efficacité de la régulation cardiovasculaire baroréflexe et non-baroréflexe a été lancée selon ce qui suit. Dans un premier temps les paramètres suivants ont été calculés:

- la durée $\Delta T_{PA}$ de la montée de $PA_f$, définie comme la différence entre le temps dudit maximum local de $PA_f$ et le temps du minimum local de $PA_f$ précédent;
- la période dite d'estimation $P_e$, définie comme la durée $\Delta T_{PA}$ de la montée de $PA_f$, multipliée par un coefficient k;
- le délai $\tau$ entre ladite montée de $PA_f$ et la montée d'

$IT_f$ qui a suivi cette montée de $PA_f$. Les paramètres $\Delta T_{PA}$, $P_e$ and $\tau$ sont illustrés dans les figures 3 et 4.

En fonction de la valeur du délai $\tau$, la réponse baroréflexe d'$IT_f$ correspondant à ladite montée de $PA_f$ a été:

- soit la montée d'$IT_f$ qui a suivi ladite montée de $PA_f$, si $\tau$ a été compris entre une limite inférieure $t_{inf}$ égale à 0s et une limite supérieure $t_{sup}$ comprise entre 10s et 20s (voir figure 3).
- soit la séquence d'$IT_f$ qui a commencé après un intervalle prédéfini $\tau_0$ compris entre 5s et 15s par rapport au début de ladite montée de $PA_f$, dans le cas où $\tau$ a été supérieur à la limite $t_{sup}$ comprise entre 10s et 20s (voir figure 4).

Dans les deux cas la durée de la réponse baroréflexe d'$IT_f$ est égale à ladite période d'estimation $P_e$.

Une fois la réponse baroréflexe d'$IT_f$ identifiée, les paramètres suivants ont été calculés:

- l'aire sous la courbe de ladite montée de $PA_f$ définie comme la somme des différences algébriques entres tous les échantillons du signal $PA_f$ durant la période d'estimation $P_e$ à partir du début de ladite montée de $PA_f$, et la valeur de la $PA_f$ au début de ladite montée de $PA_f$ (hachurée en gris dans les figures 3 et 4);
- l'aire sous la courbe de ladite réponse baroréflexe d'$IT_f$ définie comme la somme des différences algébriques entres tous les échantillons du signal $IT_f$ durant la période d'estimation $P_e$ à partir du début de ladite réponse baroréflexe d'$IT_f$, et la valeur d'$IT_f$ au début de ladite réponse baroréflexe d'$IT_f$ (hachurée en gris dans les figures 3 et 4);
- le paramètre p1 égal au rapport entre l'aire sous la courbe de ladite réponse baroréflexe d'$IT_f$ et l'aire sous la courbe de ladite montée de $PA_f$;
- l'amplitude IT+ de ladite réponse baroréflexe d'$IT_f$ définie comme la différence entre le maximum atteint par l'$IT_f$ au cours de sa montée pendant la durée de ladite réponse baroréflexe d'$IT_f$, et le niveau d'$IT_f$ au début de la montée d'$IT_f$ qui a suivi ladite montée de $PA_f$ (voir figures 3 et 4);
- le paramètre p2 égal à l'amplitude IT- de la baisse d'$IT_f$ qui a précédé la montée d'$IT_f$ qui a suivi ladite montée de $PA_f$ (voir figures 3 et 4);
- le paramètre p3 égal au rapport entre l'amplitude IT+ et l'amplitude IT-;
- le paramètre p4 égal à la différence algébrique $\Delta$IT entre la valeur d'$IT_f$ après ladite période d'estimation $P_e$ à partir du début de ladite réponse baroréflexe d'$IT_f$, et la valeur d'$IT_f$ au début de ladite réponse baroréflexe d'$IT_f$ (voir figures 3 et 4).

Selon une réalisation préférée, la surveillance de la survenue concomitante d'une inefficacité temporaire du baroréflexe cardiaque et d'une activation de la régulation cardiovasculaire non-baroréflexe a été réalisée en comparant les paramètres p1-p4 avec des seuils prédéfinis. Un index de la profondeur de l'anesthésie, notamment de la composante analgésique, nommé CARDEAN (Cardiovascular depth of anesthesia) a été calculé. Selon une réalisation préférée, l'index CARDEAN a été conçu pour prendre des valeurs entre 0 et 100, la valeur de CARDEAN étant en relation croissante avec l'allègement de l'anesthésie. Selon cette réalisation préférée, l'index CARDEAN a été conçu pour prendre des valeurs strictement supérieures à 60 si au moins les 4 conditions suivantes ont été remplies:

1) le paramètre p1 a été au-dessous d'un seuil s1 compris de préférence entre 1 et 3,
2) le paramètre p2 a été au-dessus d'un seuil s2 compris de préférence entre 40ms et 80ms,
3) le paramètre p3 a été au-dessous d'un seuil s3 compris de préférence entre 0.3 et 0.75,
4) le paramètre p4 a été au-dessous d'un seuil s4 compris de préférence entre -20 et 10. Une alarme signalant l'allègement de l'anesthésie, notamment de la composante analgésique a été émise chaque fois que l'index CARDEAN a été strictement supérieur à 60.

Les figures 3 et 4 illustrent l'algorithme de calcul de l'index CARDEAN.

La figure 3 montre le fonctionnement normal du baroréflexe cardiaque correspondant à une anesthésie adéquate. Les valeurs exactes des paramètres décrits plus haut sont les suivantes:

- amplitude de la montée de $PA_f$: 14.56mmHg
- durée $\Delta T_{PA}$ de la montée de $PA_f$: 14s
- coefficient k: 1.64
- période d'estimation $P_e$: k* $\Delta T_{PA}$ = 1.64*14=23s
- délai $\tau$ entre la montée $PA_f$ et la montée d'$IT_f$ qui a suivi cette montée de $PA_f$: 4s

Le délai $\tau$ état compris entre 0s et une limite supérieure $t_{sup}$ comprise entre 10s et 20s, la réponse baroréflexe d'$IT_f$ correspondant à ladite montée de $PA_f$ a été la montée d'$IT_f$ qui a suivi ladite montée de $PA_f$.

Les paramètres suivants ont été calculés:

- aire sous la courbe de ladite montée de $PA_f$: 231.1mmHg
- aire sous la courbe de ladite réponse baroréflexe d'$IT_f$: 2512.6 ms
- le paramètre p1 égal au rapport entre l'aire sous la courbe de ladite réponse baroréflexe d'$IT_f$ et l'aire sous la courbe de ladite montée de $PA_f$:

$$p1 = 2512.6 / 231.1 = 10.886$$

- amplitude IT+ de ladite réponse baroréflexe d'IT$_f$: 159.78 ms
- le paramètre p2 égal à l'amplitude IT- de la baisse d'IT$_f$ qui a précédé la montée d'IT$_f$ qui a suivi ladite montée de PA$_f$:

$$p2 = 106.01 \text{ ms}$$

- le paramètre p3 égal au rapport entre l'amplitude IT+ et l'amplitude IT-:

$$p3 = 159.78 / 106.01 = 1.507$$

- le paramètre p4 égal à la différence algébrique $\Delta$IT entre le niveau d'IT$_f$ à la fin de la période d'estimation hachurée en gris et le niveau d'IT$_f$ au début de ladite réponse baroréflexe d'IT$_f$:

$$p4 = 141.34 \text{ms}$$

Le paramètre p1 = 10.886 n'a pas été au-dessous du seuil s1 compris de préférence entre 1 et 3.

Le paramètre p2 = 106.01 ms a été au-dessus du seuil s2 compris de préférence entre 40ms et 80ms.

Le paramètre p3 = 1.507 n'a pas été au-dessous du seuil s3 compris de préférence entre 0.3 et 0.75.

Le paramètre p4 = 141.34 n'a pas été au-dessous du seuil s4 compris de préférence entre - 20 et 10.

Par conséquent, de toutes les 4 conditions nécessaires au déclenchement de l'alarme d'allégement d'anesthésie, uniquement la 2ème condition a été remplie. Ainsi l'index CARDEAN a été strictement inférieur à 60 et l'alarme n'a pas été déclenchée.

La figure 4 montre le fonctionnement anormal du baroréflexe cardiaque, concrétisé par la survenue concomitante d'une inefficacité temporaire du baroréflexe cardiaque et d'une activation de la régulation cardiovasculaire non-baroréflexe. Ceci correspond à un allégement de l'anesthésie, notamment de la composante analgésique. Les valeurs exactes des paramètres décrits plus haut sont les suivantes:

- amplitude de la montée de PA$_f$: 19.35 mmHg
- durée $\Delta T_{PA}$ de la montée de PA$_f$: 15s
- coefficient k: 1.53
- période d'estimation P$_e$: k* $\Delta T_{PA}$ = 1.53*15=23s
- délai $\tau$ entre la montée PA$_f$ et la montée d'IT$_f$ qui a suivi cette montée de PA$_f$: 24s

Le délai $\tau$ état supérieur à la limite t$_{sup}$ comprise entre 10s et 20s, la réponse baroréflexe d'IT$_f$ correspondant à ladite montée de PA$_f$ a été la séquence d'IT$_f$ qui a commencé après un intervalle prédéfini $\tau_0$ compris entre 5s et 15s par rapport au début de ladite montée de PA$_f$. Les paramètres suivants ont été calculés:

- aire sous la courbe de ladite montée de PA$_f$: 325.06 mmHg
- aire sous la courbe de ladite réponse baroréflexe d'IT$_f$: -823.9 ms
- le paramètre p1 égal au rapport entre l'aire sous la courbe de ladite réponse baroréflexe d'IT$_f$ et l'aire sous la courbe de ladite montée de PA$_f$:

$$p1 = -823.9 / 325.06 = -2.54$$

- amplitude IT+ de ladite réponse baroréflexe d'IT$_f$: 34.53ms
- le paramètre p2 égal à l'amplitude IT- de la baisse d'IT$_f$ qui a précédé la montée d'IT$_f$ qui a suivi ladite montée de PA$_f$:

$$p2 = 126.43 \text{ ms}$$

- le paramètre p3 égal au rapport entre l'amplitude IT+ et l'amplitude IT-:

$$p3 = 34.53 / 124.43 = 0.27$$

- le paramètre p4 égal à la différence algébrique $\Delta$IT entre le niveau d'IT$_f$ à la fin de la période d'estimation hachurée en gris et le niveau d'IT$_f$ au début de ladite réponse baroréflexe d'IT$_f$:

$$p4 = -20.36 \text{ ms}$$

La paramètre p1 = -2.54 a été au-dessous du seuil s1 compris de préférence entre 1 et 3.

Le paramètre p2 = 126.43 ms a été au-dessus du seuil s2 compris de préférence entre 40ms et 80ms.

Le paramètre p3 = 0.27 a été au-dessous du seuil s3 compris de préférence entre 0.3 et 0.75.

Le paramètre p4 = -20.36 a été au-dessous du seuil s4 compris de préférence entre -20 et 10.

Par conséquent, toutes les 4 conditions nécessaires au déclenchement de l'alarme d'allégement d'anesthésie ont été remplies. Ainsi l'index CARDEAN a été strictement supérieur à 60 et l'alarme a été déclenchée.

2/ Protocole experimental

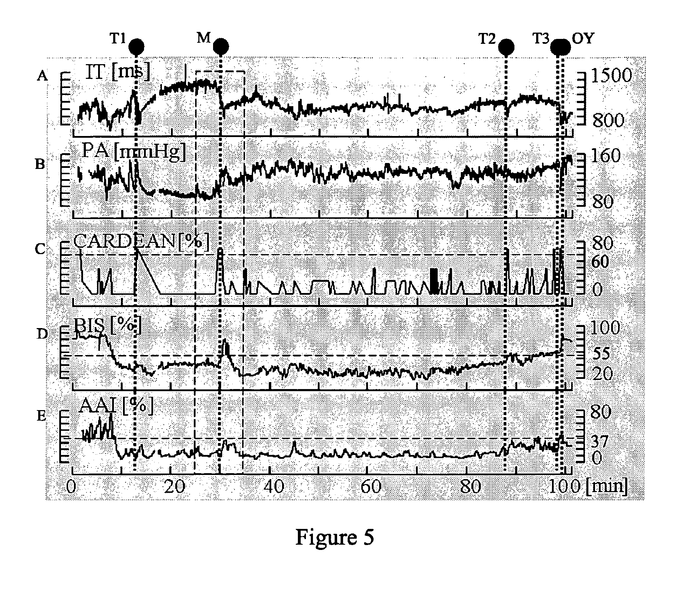

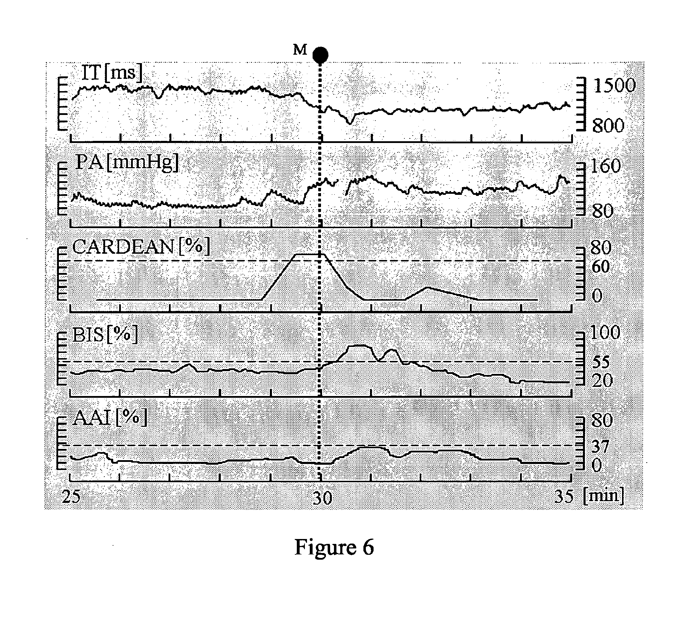

L'anesthésiste a travaillé en aveugle par rapport aux index de la profondeur de l'anesthésie dérivés de l'électroencéphalogramme (EEG), ici les index BIS et AAI. L'anesthésiste s'est guidé uniquement sur les critères cliniques habituels: hypertension, tachycardie, mouvement, toux. L'enregistrement des signaux cardiovasculaires et des index dérivés de l'EEG a commencé à t=0min (voir figure 5). L'induction de l'anesthésie a eu lieu à t=6min. A t=11min le patient était déjà anesthésié et tous les signes de Guedel étaient inhibés, à savoir le réflexe ciliaire, le réflexe cornéen, le larmoiement, le mouvement oculaire et la réponse motrice à la stimulation nociceptive. C'est à partir cet instant-ci que l'index CARDEAN a été applicable. L'intubation a eu lieu à t=12.92min. Le patient a toussé pendant environ 30s après l'intubation. La chirurgie a commencé à t=28.96min. A t=29.95min le patient a commencé à bouger de manière inattendue pendant environ une minute, empêchant le déroulement de l'opération (voir la figure 6). L'anesthésique a été arrêté à t=86.46min. Le patient a toussé à t=87.87min et à t=98.79min. Le patient a ouvert les yeux immédiatement après la stimulation verbale à t=99.14min. Le patient a été extubé dans la salle d'opération à t=99.8min.

Le seuil d'alarme pour l'index BIS a été fixé à 55%. Le seuil d'alarme pour l'index AAI a été fixé à 37%. Le seuil d'alarme pour l'index CARDEAN a été fixé à 60%.

3/ Résultats

Tous les signes de réveil (mouvement, ouverture des yeux, toux) ont été associés avec une augmentation de l'index CARDEAN selon l'invention au-dessus du seuil de 60%. A l'opposé, ces signes de réveil n'ont pas été toujours associés avec une augmentation au-dessus du seuil de 55% de l'index BIS (toux à t=12.92min) ou au-dessus du seuil de 37% de l'index AAI (toux à t=12.92min et à t=87.87min, mouvement à t=29.95min). De plus, l'augmentation de l'index CARDEAN selon l'invention a été plus précoce que l'augmentation des index BIS ou AAI (voir figure 6). En effet, le mouvement inopiné à t=29.95min, a pu être prédit par l'index CARDEAN 28s à l'avance. A l'opposé, ce mouvement à t=29.95min, n'a pas pu être prédit par les index BIS et AAI.

4/ Description des figures

Figure 1. Filtrage des suites de pression artérielle (PA). La PA non-invasive (FINAPRES 2300, Ohmeda, Englewood, CO) a été enregistrée en continu. Les suites de PA (haut) ont été obtenues battement-après-battement en calculant les pressions artérielles systoliques pour chaque cycle cardiaque. Les suites de PA ont été filtrées à l'aide d'un filtre passe-bas à réponse impulsionnelle infinie, ici de préférence un filtre de Butterworth dont la fréquence de coupure a été comprise entre 0.1Hz et 0.15Hz. Ce filtrage a donné naissance à des suites filtrées $PA_f$ (bas) dans lesquelles les variations ventilatoires (0.22 Hz) sous contrôle parasympathique ont été supprimées.

Figure 2. Filtrage des suites d'intervalles temporels (IT). L'électrocardiogramme (ECG) a été enregistré en continu. Les suites d'IT (haut) entre 2 cycles cardiaques ont été obtenues battement-après-battement en calculant les intervalles de temps (intervalles R-R) entre 2 complexes QRS de l'ECG. Les suites d'IT ont été filtrées à l'aide d'un filtre passe-bas à réponse impulsionnelle infinie, ici de préférence un filtre de Butterworth dont la fréquence de coupure a été comprise entre 0.1Hz et 0.15Hz. Ce filtrage a donné naissance à des suites filtrées $IT_f$ (bas) dans lesquelles les variations ventilatoires (0.22 Hz) sous contrôle parasympathique ont été supprimées.

Figure 3. Fonctionnement normal du baroréflexe cardiaque. Suites d'intervalles temporels filtrés $IT_f$ (haut) et de pressions artérielles filtrées $PA_f$ (bas) selon le procédé évoqué dans les figures 1 et 2. Au moment t=0 une montée de $PA_f$ a lieu (zone hachurée en gris). Cette montée de $PA_f$ est suivie dans un délai $\tau$ (ici environ 4s) d'une montée de l'$IT_f$ Le délai $\tau$ étant inférieur à une limite comprise entre 10s et 20s, la réponse baroréflexe d'$IT_f$ correspondant à ladite montée de $PA_f$ a été la montée d'$IT_f$ qui a suivi ladite montée de $PA_f$ (zone hachurée en gris). $\Delta T_{PA}$: délai de ladite montée de $PA_f$. $P_e$: période dite d'estimation égale au délai $\Delta T_{PA}$ multiplié avec un coefficient k. IT+: amplitude de ladite réponse baroréflexe d'$IT_f$. IT-: amplitude de la baisse d'$IT_f$ qui a précédé ladite montée d'$IT_f$. $\Delta IT$: différence algébrique entre le niveau d'$IT_f$ à la fin de la période d'estimation hachurée en gris et le niveau d'$IT_f$ au début de ladite réponse baroréflexe d'$IT_f$. Cette figure montre un fonctionnement efficace du baroréflexe cardiaque car ladite réponse baroréflexe a été rapide et de grande amplitude. Dans ce cas, la profondeur de l'anesthésie est considérée comme adéquate par l'index CARDEAN selon l'invention. La profondeur de l'anesthésie était adéquate également selon les signes cliniques classiques: absence de mouvement spontané, absence de réponse motrice à un stimulus douloureux, absence des réflexes ciliaire et cornéen, pupilles centrées et fixes.

Figure 4. Fonctionnement anormal du baroréflexe cardiaque. Suites d'intervalles temporels filtrés $IT_f$ (haut) et de pressions artérielles filtrées $PA_f$ (bas) selon le procédé évoqué dans les figures 1 et 2. Au moment t=0 une montée de $PA_f$ a lieu (zone hachurée en gris). Cette montée de $PA_f$ est suivie dans un délai $\tau$ (ici environ 24s) d'une montée de l'$IT_f$. Le délai $\tau$ étant supérieur à une limite comprise entre 10s et 20s, la réponse baroréflexe d'$IT_f$ correspondant à ladite montée de $PA_f$ a été la séquence d'$IT_f$ qui a commencé après un intervalle prédéfini $\tau_0$ com-

pris entre 5s et 15s par rapport au début de ladite montée de PA$_f$, (zone hachurée en gris). $\Delta T_{PA}$: délai de ladite montée de PA$_f$ P$_e$: période dite d'estimation égale au délai $\Delta T_{PA}$ multiplié avec un coefficient k. IT+: amplitude de ladite réponse baroréflexe d'IT$_f$. IT-: amplitude de la baisse d'IT$_f$ qui a précédé ladite montée de l'IT$_f$. $\Delta$IT: différence algébrique entre le niveau d'IT$_f$ à la fin de la période d'estimation hachurée en gris et le niveau d'IT$_f$ au début de ladite réponse baroréflexe d'IT$_f$ Cette figure montre la survenue concomitante d'une inefficacité temporaire du baroréflexe cardiaque et d'une activation de la régulation cardiovasculaire non-baroréflexe. En effet, ladite réponse baroréflexe a été retardée et de faible amplitude, laissant la place à une tachycardie non-baroréflexe. Dans ce cas, la profondeur de l'anesthésie est considérée comme inadéquate par l'index CARDEAN selon l'invention. La profondeur de l'anesthésie s'est avérée être inadéquate car un mouvement inopiné du patient a eu lieu environ 28s plus tard. L'index CARDEAN selon l'invention a pu prédire ce "réveil" inopiné.

Figure 5. Comparaison de l'index CARDEAN selon l'invention avec les index BIS et AAI. Enregistrement en continu des intervalles temporels IT (A), des pressions artérielles PA (B) et des index CARDEAN (C), BIS (D) et AAI (AAI) au cours d'une intervention chirurgicale sous anesthésie générale. L'induction de l'anesthésie a eu lieu à t=6min. L'intubation a eu lieu à t=12.92min. Le patient a toussé pendant environ 30s après l'intubation (trait en pointillés:"T1"). La chirurgie a commencé à t=28.96min. A t=29.95min le patient a commencé à bouger de manière inattendue pendant environ une minute, empêchant le déroulement de l'opération (trait en pointillés:"M"). La période entre t=25min et t=35min (rectangle en pointillés) est agrandie dans la figure 6. L'anesthésique a été arrêté à t=86.46min. Le patient a toussé à t=87.87min (trait en pointillés:"T2") et à t=98.79min (trait en pointillés: "T3 "). Le patient a ouvert les yeux immédiatement après la stimulation verbale à t=99.14min (trait rouge en pointillés: "OY"). Le patient a été extubé dans la salle d'opération à t=99.8min. Les seuils d'alarme pour les index BIS, AAI et CARDEAN ont été fixés respectivement à 55%, 37% et 60% (traits horizontaux en pointillés). L'index CARDEAN a franchi le seuil d'alarme pour tous les signes de réveil (mouvement, ouverture des yeux, toux). A l'opposé, les index BIS et AAI n'ont pas franchi le seuil d'alarme pour les signes de réveil suivants: toux à t=12.92min (BIS et AAI), toux à t=87.87min (AAI) et, mouvement à t=29.95min (AAI).

Figure 6. Comparaison de l'index CARDEAN selon l'invention avec les index BIS et AAI. Agrandissement de la figure 6 entre t=25min et t=35min montrant plus en détail le mouvement qui a eu lieu à t=29.95min (trait en pointillés: "M"). Les seuils d'alarme pour les index BIS, AAI et CARDEAN ont été fixés respectivement à 55%, 37% et 60% (traits horizontaux en pointillés). Le seul index qui a franchi le seuil d'alarme avant le mouvement a été l'index CARDEAN. Le mouvement inopiné à t=29.95min a pu être prédit par l'index CARDEAN environ 28s à l'avance. A l'opposé, ce mouvement à t=29.95min, n'a pas pu être prédit par les index BIS et AAI.

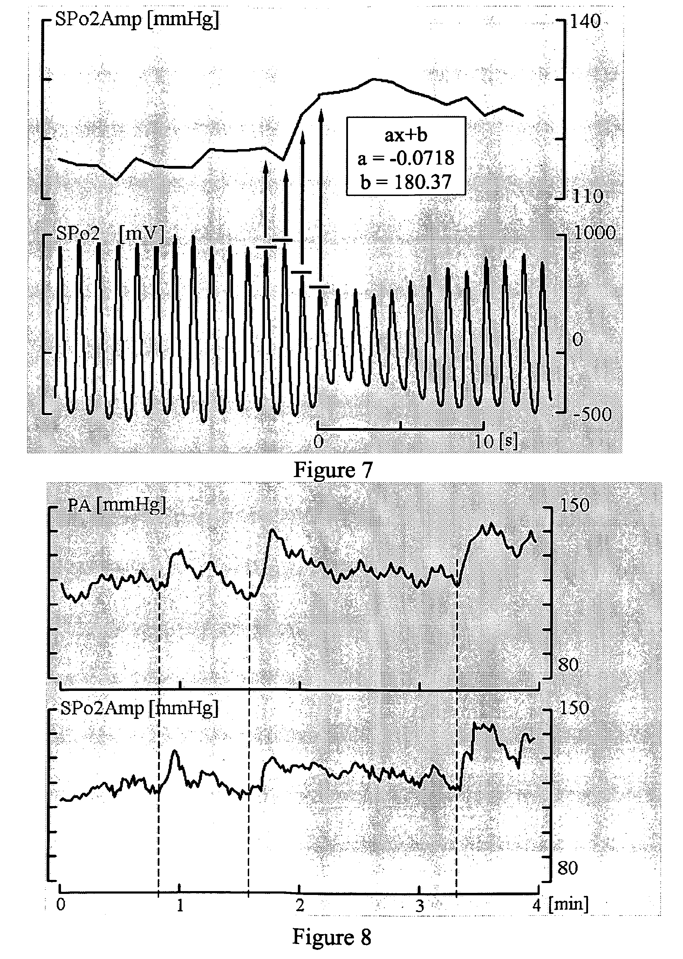

Figure 7. Transformation de l'onde de la saturation en oxygène du sang (Sp02) en continu (bas) dans une suite des valeurs (SpO2Amp) (haut) qui approche la suite des pressions artérielles (PA) battement par battement. La méthode consiste à détecter les maximums du signal SpO2 pour chaque cycle cardiaque (traits horizontaux) et à transformer l'amplitude de chaque maximum à l'aide d'un opérateur linéaire du 1$^{er}$ degré (ax + b). Les coefficients de l'opérateur linéaire (a et b) ont été calculés à partir des valeurs intermittentes de PA systolique mesurées à l'aide d'un brassard à tension.

Figure 8. Comparaison des suites de PA systolique (haut) obtenues à l'aide d'un appareil de mesure non-invasif (FINAPRES 2300, Ohmeda, Englewood, CO) avec les suites des PA systolique (Sp02Amp) (bas) obtenues indirectement à partir de l'onde de la saturation en oxygène du sang (SpO2) en continu (voir figure 7). Les 2 suites de PA présentent des variations similaires. En outre, les débuts des montées de PA sont concomitants sur les 2 suites de PA (traits en pointillés).

## Revendications

1.  Dispositif pour la prédiction d'évènements médicaux anormaux et/ou d'aide au diagnostic et/ou de monitorage **caractérisé en ce qu'**il comprend des moyens de détection, en continu et en temps réel, de la survenue concomitante d'une inefficacité temporaire du baroréflexe cardiaque et d'une activation de la régulation cardiovasculaire non-baroréflexe et **en ce qu'**il comprend essentiellement :

    a) des moyens de mesure en continu (battement-après-battement) des intervalles temporels entre 2 cycles cardiaques consécutifs (IT) et de la pression artérielle (PA) ;
    b) un filtre passe-bas pour filtrer des suites battement-après-battement des intervalles temporels IT et des suites de PA précédemment mesurées, pour éliminer les variations rapides sous contrôle parasympathique, à la fréquence ventilatoire supérieure ou égale à un seuil compris entre 0,1 et 0.15 Hz, ce filtrage donnant naissance à des suites d'IT et de PA filtrées IT$_f$, respectivement PA$_f$;

c) des moyens de surveillance de la survenue concomitante d'une inefficacité temporaire du baroréflexe cardiaque et d'une activation de la régulation cardiovasculaire non-baroréflexe au travers de la manifestation d'événements choisis dans le groupe de séquences suivantes :

■ augmentation de PA$_f$ / délai / augmentation réduite de IT$_f$;
■ augmentation de PA$_f$ / délai / augmentation retardée de IT$_f$;
■ augmentation de PA$_f$ / délai / diminution de IT$_f$;
■ ainsi que la combinaison d'au moins deux de ces séquences;

d) et au moins une alarme pour avertir de la survenue concomitante d'une inefficacité temporaire du baroréflexe cardiaque et d'une activation de la régulation cardiovasculaire non-baroréflexe.

2. Dispositif selon la revendication 1, **caractérisé en ce qu'**il comprend des moyens pour calculer les intervalles temporels IT soit à partir des intervalles RR, c'est-à-dire des intervalles de temps entre deux complexes QRS de l'ECG, soit à partir des intervalles entre deux points caractéristiques (débuts de montée ou maximums ou autres) de la PA en continu ou de la saturation en oxygène du sang (SpO2) en continu;

3. Dispositif selon la revendication 1 ou 2, **caractérisé en ce que** la PA est choisie dans le groupe de signaux comprenant: la pression artérielle systolique (PAS) du patient, la pression artérielle diastolique (PAD) du patient, la pression artérielle moyenne (PAM) du patient, la PAS étant préférée.

4. Dispositif selon l'une des revendications 1 à 3, **caractérisé en ce qu'**il comprend des moyens de mesure de la PA en continu soit par mesure directe invasive ou non-invasive, soit par mesure indirecte de préférence à partir de la SpO2 en continu, calibrée à l'aide des valeurs intermittentes de PA obtenues de préférence à l'aide d'un brassard à tension.

5. Dispositif selon l'une des revendications 1 à 4, **caractérisé en ce que** dans le cas où les suites de PA en continu sont obtenues de manière indirecte à l'aide de la SpO2 en continu calibrée avec les valeurs intermittentes de PA, il comprend :

a1) des moyens de calcul de la suite des maximums du signal SpO2 lors de chaque cycle cardiaque ;
a2) des moyens d'inversion de la suite obtenue à l'étape a1) par soustraction d'une constante strictement supérieure à l'amplitude maximale du signal SP02 ;
a3) des moyens de calibrage de la suite obtenue à l'étape a2) en unités de pression en appliquant un opérateur linéaire du 1$^{er}$ degré à la suite obtenue à l'étape a2), les coefficients de cet opérateur étant obtenus à partir des valeurs intermittentes de PA.

6. Dispositif selon l'une des revendications 1 à 5, **caractérisé en ce qu'**il comprend au moins un filtre à réponse impulsionnelle infinie (RII) ou à réponse impulsionnelle finie, le filtre de type RII étant préféré, pour permettre que le filtrage passe-bas des suites d'IT et de PA soit réalisé en temps réel.

7. Dispositif selon l'une des revendications 1 à 6, **caractérisé en ce qu'**il comprend au moins un écran pour qu'au moins l'un des signaux suivants soit affiché : les suites d'IT et de PA et les suites filtrées d'IT$_f$ et PA$_f$.

8. Dispositif selon l'une des revendications de 1 à 7, **caractérisé en ce que** les moyens (c) de surveillance de la survenue concomitante d'une inefficacité temporaire du baroréflexe cardiaque et d'une activation de la régulation cardiovasculaire non-baroréflexe, mettent en oeuvre au moins quatre paramètres suivants :

p1) le rapport entre l'aire sous la courbe d'une séquence d'IT$_f$ dite réponse baroréflexe d'IT$_f$ et l'aire sous la courbe de la montée de PA$_f$ qui a provoqué ladite réponse baroréflexe d'IT$_f$,
p2) l'amplitude de la baisse de l'IT$_f$ qui a précédé la montée d'IT$_f$ qui suit ladite montée de PA$_f$,
p3) le rapport entre l'amplitude de ladite réponse baroréflexe d'IT$_f$ et l'amplitude de ladite baisse d'IT$_f$,
p4) la différence algébrique $\Delta$IT entre la valeur d'IT$_f$ après une période de temps dite d'estimation à partir du début de ladite réponse baroréflexe d'IT$_f$, et la valeur d'IT$_f$ au début de ladite réponse baroréflexe d'IT$_f$,
ainsi que, toute combinaison pondérée ou pas des paramètres précédents.

9. Dispositif selon la revendication 8, **caractérisé en ce que** ladite réponse baroréflexe d'IT$_f$ provoquée par ladite montée de PA$_f$, est :

● soit la montée d'IT$_f$ qui suit ladite montée de PA$_f$, si ladite montée d'IT$_f$ commence dans un intervalle de temps compris entre une limite inférieure égale à 0s et une limite supérieure comprise entre 10s et 20s par rapport au début de ladite montée de PA$_f$;

● soit la séquence d'$IT_f$ qui commence après un intervalle compris entre 5s et 15s par rapport au début de ladite montée de $PA_f$, dans le cas où la montée d'$IT_f$ qui suit ladite montée de $PA_f$, commence après ladite limite supérieure comprise entre 10s et 20s par rapport au début de ladite montée de $PA_f$;

dans les deux cas, la durée de la réponse baroréflexe d'$IT_f$ étant égale à ladite période d'estimation ; l'amplitude de ladite réponse baroréflexe d'$IT_f$ étant la différence entre le maximum atteint par l'$IT_f$ au cours de sa montée pendant la durée de ladite réponse baroréflexe d'$IT_f$, et le niveau d'$IT_f$ au début de la montée d'$IT_f$ qui suit ladite montée de $PA_f$.

10. Dispositif selon l'une des revendications 8 ou 9, **caractérisé en ce que** les seules montées de $PA_f$ prises en considération, sont celles dont l'amplitude est supérieure à un seuil de pression compris entre 1mmHg et 5mmHg, de préférence entre 2mmHg et 4 mmHg, et mieux encore entre 2 mmHg et 3 mmHg, les autres montées de $PA_f$ étant négligées.

11. Dispositif selon l'une des revendications 8 à 10, **caractérisé en ce que** la période dite d'estimation est égale à la durée de ladite montée de $PA_f$ multipliée par un coefficient compris entre 0.5 et 2, de préférence entre 1 et 1.8.

12. Dispositif selon l'une des revendications 8 à 11, **caractérisé en ce qu'**il comprend des moyens de calcul desdites aires sous les courbes de la réponse baroréflexe d'$IT_f$ et de la montée de $PA_f$ sont calculées entre un moment dit de référence et pendant une certaine durée en réalisant pour chaque échantillon de ladite courbe au cours de ladite durée à partir dudit moment de référence, la somme des différences algébriques entre la valeur de l'échantillon à un moment donné et la valeur de la courbe audit moment de référence.

13. Dispositif selon l'une des revendications 1 à 12, **caractérisé en ce qu'**il comprend au moins une alarme destinée à prédire tout allègement programmé ou intempestif de l'anesthésie, notamment de la composante analgésique, et **en ce qu'**il comprend des moyens d'affichage de l'alarme destinée à avertir de la survenue concomitante d'une inefficacité temporaire du baroréflexe cardiaque et d'une activation de la régulation cardiovasculaire non-baroréflexe, par affichage, en continu et en temps réel, d'un index prédictif exprimant la profondeur cardiovasculaire de l'anesthésie.

**Claims**

1. Device for the prediction of abnormal medical events and/or for assisting diagnosis and/or for monitoring, **characterized in that** it comprises means for detecting, continuously and in real time, the concomitant occurrence of a temporary inefficiency of the cardiac baro-reflex and of an activation of the non-baro-reflex cardiovascular regulation, and **in that** it comprises essentially:

a) means for continuous measurement (beat-by-beat) of the time intervals between 2 consecutive cardiac cycles (IT) and of the arterial pressure (AP);
b) a low-pass filter to filter beat-by-beat series of the previously measured time intervals IT and series of the previously measured AP, in order to eliminate the rapid variations under parasympathetic control, at the upper respiratory frequency or the frequency equal to a threshold in the range between 0.1 and 0.15 Hz, this filtering giving rise to filtered series 1Tf and APf of IT and of AP, respectively;
c) means for surveillance of the concomitant occurrence of a temporary inefficiency of the cardiac baro-reflex and of an activation of the non-baro-reflex cardiovascular regulation via the manifestation of events chosen from the following group of sequences:

■ increase in APf/time period/reduced increase in ITf;
■ increase in APf/time period/delayed increase in ITf;
■ increase in APf/time period/decrease in ITf;
■ and also the combination of at least two of these sequences;

d) and at least an alarm for warning of the concomitant occurrence of a temporary inefficiency of the cardiac baro-reflex and of an activation of the non-baro-reflex cardiovascular regulation according to step c).

2. Device according to claim 1, **characterized in that** it comprises means for calculating the time intervals IT either from the intervals RR, in other words from the intervals of time between two QRS complexes from the ECG, or from the intervals between two points that are characteristic (start points of rise or maxima or others) of the continuous AP or of the continuous oxygen saturation level of the blood (Sp02).

3. Device according to claim 1 or 2, **characterized in that** the AP is chosen from the group of signals comprising: the systolic arterial pressure (SAP) of the patient, the diastolic arterial pressure (DAP) of the patient, the mean arterial pressure (MAP) of the patient, the SAP being preferred.

4. Device according to any of the claims 1 to 3, **characterized in that** it comprises means for continuous measurement of AP, either by direct invasive or non-invasive measurement, or by indirect measurement preferably using the continuous Sp02, calibrated by means of the intermittent values of AP preferably obtained by means of a blood-pressure armband.

5. Device according to any of the claims I to 4, **characterized in that**, in the case where the continuous series of AP are obtained in an indirect manner by means of the continuous Sp02 calibrated with the intermittent values of AP, it comprises:

> a1) means for calculating the series of the maxima of the Sp02 signal during each cardiac cycle;
> a2) means for inverting the series obtained at step a1) by subtracting from a constant strictly higher than the maximum amplitude of the Sp02 signal;
> a3) means for calibrating the series obtained at step a2) in units of pressure by applying a linear operator of the $1^{st}$ degree to the series obtained at step a2), the coefficients of this operator being obtained from the intermittent values of AP.

6. Device according to any of the claims I to 5, **characterized in that** it comprises at least one filter with infinite pulse response (RII) or with finite pulse response, the filter of the RII type being preferred, to make it possible the low-pass filtering of the series of IT and of AP be carried out in real time.

7. Device according to any of the claims I to 6, **characterized in that** it comprises at least one screen in order that at least one of the following signals be displayed: the series of IT and af AP and the filtered series of ITf and APf.

8. Device according to any of the claims 1 to 7, **characterized in that** the means c) for surveillance of the concomitant occurrence of a temporary inefficiency of the cardiac baro-reflex and of an activation of the non-baro-reflex cardiovascular regulation, use at least four of the following parameters:

> p1) the ratio between the area under the curve of a sequence of ITf called baro-reflex response of ITf and the area under the curve of the rise in APf which has caused said baro-reflex response of ITf,
> p2) the amplitude of the fall in ITf which has preceded the rise in ITf that follows said rise in APf,
p3) the ratio between the amplitude of said baro-reflex response of ITf and the amplitude of said fall in ITf,
p4) the algebraic difference [Delta]IT between the value of ITf after a period of time referred to as estimation time starting from the beginning of said baro-reflex response of ITf and the value of ITf at the start of said baro-reflex response of ITf,

and also any combination, weighted or not, of the preceding parameters.

9. Device according to claim 8, **characterized in that** said baro-reflex response of ITf, caused by said rise in APf, is:

> ● either the rise in ITf that follows said rise in APf if said rise in ITf begins within an interval of time included between a lower limit equal to 0 s and an upper limit included between 10 s and 20 s with respect to the start of said rise in APf;
> ● or the sequence of ITf which begins after an interval of time included between 5 s and 15 s with respect to the start of said rise in APf, in the case where the rise in ITf that follows said rise in APf begins after said upper limit in the range between 10 s and 20 s with respect to the start of said rise in APf;
> in both cases, the duration of the baro-reflex response of ITf being equal to said estimation period; the amplitude of said baro-reflex response of ITf being the difference between the maximum reached by the ITf in the course of its rise over the duration of said baro-reflex response of ITf and the level of ITf at the start of the rise in ITf that follows said rise in APf.

10. Device according to claim 8 or 9, **characterized in that** the only rises in APf taken into consideration are those whose amplitude is higher than a pressure threshold in the range between 1 mmHg and 5 mmHg, preferably between 2 mmHg and 4 mmHg, or even better between 2 mmHg and 3 mmHg, the other rises in APf being neglected.

11. Device according to any of the claims 8 to 10, **characterized in that** the period called estimation period is equal to the duration of said rise in APf multiplied by a coefficient in the range between 0.5 and 2, preferably between 1 and 1.8.

12. Device according to any of the claims 8 to 11, **characterized in that** it comprises means for calculating the said areas under the curves of the baro-reflex response of ITf and of the rise of Apf, calculated between a time called reference time and during said estimation period by performing, for each sample of said curve in the course of said estimation period starting from said reference time, the sum of the algebraic differences between the value of the sample at a given moment and the value of the curve at said reference time.

13. Device according to any of the claims 8 to 11, **characterized in that** it comprises at least one alarm designed to predict any lightening, programmed or inopportune, of the anesthesia, notably of the analgesic component, and **in that** it comprises means for displaying the alarm designed to warn of the concomitant occurrence of a temporary inefficiency of the cardiac baro-reflex and of an activation of the non-baro-reflex cardiovascular regulation, by continuously displaying in real time a predictive index expressing the cardiovascular depth of the anesthesia.

**Patentansprüche**

1. Vorrichtung zur Vorhersage von abweichenden medizinischen Ereignissen und/oder zur Diagnosehilfe und/oder Überwachung, **dadurch gekennzeichnet, dass** sie Mittel zur kontinuierlichen Erkennung in Echtzeit des plötzlichen Auftretens einer temporären Unwirksamkeit des Herz-Barorezeptor-Reflexes und einer Aktivierung der nicht baroreflexen kardiovaskulären Regulation umfasst, und dadurch, dass sie im Wesentlichen Folgendes umfasst:

a) Mittel zur kontinuierlichen Messung (Beat-to-beat) der Zeitintervalle zwischen 2 aufeinanderfolgenden Herzzyklen (IT) und des arteriellen Drucks (AD);
b) einen Tiefpassfilter zur Filterung von vorab gemessenen Beat-to-beat-Sequenzen der Zeitintervalle IT und AD-Sequenzen, um die schnellen Variationen unter parasympathischer Kontrolle zu eliminieren, bei einer Atemfrequenz, die gleich oder höher einem Schwellenwert zwischen 0,1 und 0,15 Hz ist, wobei diese Filterung gefilterte Zeitinterfall-Sequenzen und AD-Sequenzen, $IT_f$ beziehungsweise $AD_f$ hervorbringt;
c) Mittel zur Überwachung des plötzlichen Auftretens einer temporären Unwirksamkeit des Herz-Barorezeptor-Reflexes und einer Aktivierung der nicht baroreflexen kardiovaskulären Regulation mittels Aufzeigen von Ereignissen, die in der Gruppe folgender Sequenzen ausgewählt wurden:

■ Erhöhung von $AD_f$ / Zeitspanne / reduzierte Erhöhung von $IT_f$;
■ Erhöhung von $AD_f$ / Zeitspanne / verzögerte Erhöhung von $IT_f$;
■ Erhöhung von $AD_f$ / Zeitspanne / Verringerung von $IT_f$;
■ sowie die Kombination von mindestens zwei dieser Sequenzen;

d) und mindestens ein Alarmsignal, um das plötzliche Auftreten einer temporären Unwirksamkeit des Herz-Barorezeptor-Reflexes und einer Aktivierung der nicht baroreflexen kardiovaskulären Regulation zu melden.

2. Vorrichtung nach Anspruch 1, **dadurch gekennzeichnet, dass** sie Mittel zur Berechnung der Zeitintervalle IT umfasst, entweder ausgehend von den RR-Intervallen, das heißt, Zeitintervalle zwischen zwei QRS-Komplexen des EKG, oder ausgehend von den Intervallen zwischen zwei charakteristischen Punkten (Anstiegsbeginn oder Höchstwerte oder andere) des kontinuierlichen AD oder der kontinuierlichen Sauerstoffsättigung des Blutes (SpO2).

3. Vorrichtung nach Anspruch 1 oder 2, **dadurch gekennzeichnet, dass** der AD in der Signalgruppe ausgewählt wird, die Folgendes umfasst: den systolischen arteriellen Druck (SAD) des Patienten, den diastolischen arteriellen Druck (DAD) des Patienten, den mittleren arteriellen Druck (MAD) des Patienten, wobei der SAD bevorzugt wird.

4. Vorrichtung nach einem der Ansprüche 1 bis 3, **dadurch gekennzeichnet, dass** sie Mittel zur kontinuierlichen Messung des AD entweder durch direkte invasive oder nicht-invasive Messung oder durch indirekte Messung vorzugsweise anhand der kontinuierlichen SpO2 umfasst, kalibriert mit Hilfe der intermittierenden AD-Werte, die vorzugsweise mit Hilfe einer Druck-Armbinde erhalten werden.

5. Vorrichtung nach einem der Ansprüche 1 bis 4, **dadurch gekennzeichnet, dass** sie für den Fall, dass die kontinuierlichen AD-Sequenzen auf indirekte Weise mit Hilfe der kontinuierlichen SpO2, kalibriert mit Hilfe der intermittierenden AD-Werte, erhalten werden, Folgendes umfasst:

a1) Mittel zur Berechnung der Sequenz der Höchstwerte des SpO2-Signals bei jedem Herzzyklus;
a2) Mittel zur Umkehrung der in Schritt a1) erhaltenen Sequenz durch Subtraktion einer Konstante, die strikt über der Höchstamplitude des SP02-Signals liegen muss;
a3) Mittel zur Kalibrierung der in Schritt a2) erhaltenen Sequenz in Druckeinheiten unter Anwendung eines linearen Operators 1. Grades bei der in Schritt a2) erhaltenen Sequenz, wobei die Koeffizienten dieses Operators durch intermittierende AD-Werte erhalten werden.

**6.** Vorrichtung nach einem der Ansprüche 1 bis 5, **dadurch gekennzeichnet, dass** sie mindestens einen Filter mit unendlicher Impulsantwort (IIR) oder mit endlicher Impulsantwort umfasst, vorzugsweise den Filter vom Typ IIR, um zu ermöglichen, dass die Tiefpassfilterung der IT-Sequenzen und AD-Sequenzen in Echtzeit erfolgt.

**7.** Vorrichtung nach einem der Ansprüche 1 bis 6, **dadurch gekennzeichnet, dass** sie mindestens einen Bildschirm umfasst, damit zumindest eines der folgenden Signale angezeigt wird: die IT- und AD-Sequenzen und die gefilterten Sequenzen $IT_f$ und $AD_f$.

**8.** Vorrichtung nach einem der Ansprüche 1 bis 7, **dadurch gekennzeichnet, dass** die Mittel (c) zur Überwachung des plötzlichen Auftretens einer temporären Unwirksamkeit des Herz-Barorezeptor-Reflexes und einer Aktivierung der nicht baroreflexen kardiovaskulären Regulation mindestens vier folgende Parameter aufstellen:

p1) das Verhältnis zwischen der Fläche unter der Kurve von einer $IT_f$-Sequenz, der sogenannten Baroreflex-Antwort von $IT_f$, und der Fläche unter der Kurve des Ansteigens von $AD_f$, das die besagte Baroreflex-Antwort von $IT_f$ verursacht hat,
p2) die Amplitude des Sinkens von $IT_f$, das dem Ansteigen von $IT_f$ vorangegangen ist, das auf das besagte Ansteigen von $AD_f$ folgt,
p3) das Verhältnis zwischen der Amplitude der besagten Baroreflex-Antwort von $IT_f$ und der Amplitude des besagten Sinkens von $IT_f$,
p4) die algebraische Differenz $\Delta IT$ zwischen dem Wert $IT_f$ nach einem Zeitraum, dem sogenannten Schätzzeitraum, ab dem Beginn der besagten Baroreflex-Antwort von $IT_f$ und dem Wert $IT_f$ zu Beginn der besagten Baroreflex-Antwort von $IT_f$,
sowie jegliche gewichtete oder nicht gewichtete Kombination der vorstehenden Parameter.

**9.** Vorrichtung nach Anspruch 8, **dadurch gekennzeichnet, dass** es sich bei der besagten Baroreflex-Antwort von $IT_f$, die durch das besagte Ansteigen von $AD_f$ verursacht wurde, handelt um:

● entweder um das Ansteigen von $IT_f$, das auf das besagte Ansteigen von $AD_f$ folgt, wenn das besagte Ansteigen von $IT_f$ in einem Zeitintervall beginnt, der zwischen einer Untergrenze von gleich 0 s und einer Obergrenze zwischen einschließlich 10 s und 20 s in Bezug auf den Beginn des besagten Ansteigens von $AD_f$ liegt,
● oder die Sequenz von $IT_f$, die nach einem Intervall zwischen einschließlich 5 s und 15 s in Bezug auf den Beginn des besagten Ansteigens von $AD_f$ beginnt, für den Fall, dass das Ansteigen von $IT_f$, das auf das besagte Ansteigen von $AD_f$ folgt, nach der besagten Obergrenze zwischen einschließlich 10 s und 20 s in Bezug auf den Beginn des besagten Ansteigens von $AD_f$ beginnt,
wobei in beiden Fällen die Dauer der Baroreflex-Antwort von $IT_f$ gleich dem besagten Schätzzeitraum ist; die Amplitude der besagten Baroreflex-Antwort von $IT_f$ die Differenz zwischen dem Höchstwert von $IT_f$, der im Laufe seines Ansteigens während der Dauer der besagten Baroreflex-Antwort $IT_f$ erreicht wurde, und dem Niveau von $IT_f$ zu Beginn des Ansteigens von $IT_f$, das auf das besagte Ansteigen von $AD_f$ folgt, ist.

**10.** Vorrichtung nach einem der Ansprüche 8 oder 9, **dadurch gekennzeichnet, dass** die einzigen berücksichtigten Anstiege von $AD_f$ jene sind, bei denen die Amplitude über einem Druckschwellenwert liegt, der zwischen 1 mmHg und 5 mmHg umfasst, vorzugsweise zwischen 2 mmHg und 4 mmHg, und besser noch zwischen 2 mmHg und 3 mmHg, wobei die anderen Anstiege von $AD_f$ vernachlässigt werden.

**11.** Vorrichtung nach einem der Ansprüche 8 bis 10, **dadurch gekennzeichnet, dass** der sogenannte Schätzzeitraum gleich der Dauer des besagten Ansteigens von $AD_f$ ist, multipliziert mit einem Koeffizienten zwischen einschließlich 0,5 und 2, vorzugsweise zwischen 1 und 1,8.

**12.** Vorrichtung nach einem der Ansprüche 8 bis 11, **dadurch gekennzeichnet, dass** sie Mittel zur Berechnung der genannten Flächen unter den Kurven der Baroreflex-Antwort von $IT_f$ und dem Ansteigen von $AD_f$ umfasst, die zwischen einem bestimmten Referenzmoment und während einer bestimmten Dauer berechnet werden, indem für jede Probe der besagten Kurve während der Verlaufsdauer ab dem besagten Referenzmoment die Summe aus den algebraischen Differenzen zwischen dem Wert der Probe zu einem gegebenen Moment und dem Wert der Kurve in dem besagten Referenzmoment gebildet wird.

**13.** Vorrichtung nach einem der Ansprüche 1 bis 12, **dadurch gekennzeichnet, dass** sie mindestens ein Alarmsignal umfasst, das dazu bestimmt ist, jegliche programmierte oder unzeitgemäße Verringerung der Anästhesie, insbesondere des analgetischen Bestandteils, vorherzusagen, und dadurch, dass sie Mittel zum Anzeigen des Alarms umfasst, dazu bestimmt, das plötzliche Auftreten einer temporären Unwirksamkeit des Herz-Barorezeptor-Reflexes und einer Aktivierung der nicht baroreflexen kardiovaskulären Regulation zu melden, durch Anzeigen sowohl kontinuierlich als auch in Echtzeit eines Vorhersageindexes, der die kardiovaskuläre Tiefe der Anästhesie angibt.

Figure 1

Figure 2

Figure 3

Figure 4

Figure 5

Figure 6

Figure 7

Figure 8

## RÉFÉRENCES CITÉES DANS LA DESCRIPTION

**Documents brevets cités dans la description**

- WO 2004034897 A
- US 5437285 B
- US 5419338 B
- US 5967995 B
- WO A9503739 A
- US 5439004 B
- FR 2747027 B
- US 5372140 A
- US 6685649 B
- US 4907597 A

**Littérature non-brevet citée dans la description**

- **GRATZE et al.** *Computers in Biology and Medicine,* 1998, vol. 28, 121-142
- **LEGRAMENTE et al.** *Circulation,* 1999, vol. 99, 1761-1766
- **RANTANEN, M. et al.** *Anesthesiology,* 2004, vol. 101, A559
- **STRUYS, M. M. et al.** *Anesthesiology,* 2002, vol. 96, 803-816